# EUROPEAN PATENT APPLICATION

(11) **EP 4 668 280 A1**
(43) Date of publication of application: **24.12.2025**
(21) Application number: 24843562.0
(22) Date of filing: 19.07.2024
(51) Int. Cl.: G16C 20/70, G16C 20/40, G16C 20/90, G16C 20/80, G16C 20/30, G16C 20/10, G06F 16/332, G06F 16/338, G06N 5/022, H04L 51/02

(54) **METHOD AND SYSTEM FOR GENERATING ANSWERS**

(30) Priority: 19.07.2023 KR 20230093645
(71) Applicant: LG Management Development Institute Co., Ltd., Seoul 07336 (KR)
(72) Inventor: HORMAZABAL, Rodrigo, Incheon 21022 (KR); BERTENS, Paul, Seoul 07788 (KR); HAN, Se Hui, Seoul 07665 (KR)
(74) Representative: BCKIP Part mbB
(86) International application number: PCT/KR2024/010503
(87) International publication number: WO 2025/018851

(57) **Abstract**

The present invention relates to an answer generation method and system. More specifically, according to the present invention, an answer generation method performed by cooperation of a memory and at least one processor includes specifying an analysis target document, extracting a plurality of content from the document, storing the plurality of content extracted from the document in the memory, receiving a user query from a user terminal, specifying specific content related to the user query among the plurality of content stored in the memory, processing the specific content as input to a pre-trained chemical reaction prediction model, and generating an answer to the user query using output data of the chemical reaction prediction model.

## Description

### [Technical Field]

Various embodiments disclosed in the present document relate to an answer generation method and system, and provides an answer generation method and system using a generative model or a foundation model.

### [Background Art]

Recently, there has been a rapid increase in cases where artificial intelligence, especially deep learning, which extracts data characteristics using deep neural network structures, has achieved excellent results in various fields such as voice recognition, image recognition, natural language processing, and autonomous driving.

With the development of such deep learning technology, generative artificial intelligence (generative AI) technology is recently receiving attention. More specifically, generative AI models may generate new data in various forms, such as text, images, and voices, from given data, and provide different levels of application potential from simply classifying or predicting existing data.

In other words, as sentences, images, voices, etc., that were previously created by humans may be automatically generated using generative artificial intelligence models, services (e.g., ChatGPT) using generative artificial intelligence has shown greater activity and accuracy than the existing chatbot services and is receiving great attention worldwide.

Meanwhile, attempts are continuously being made to solve various scientific problems in the field of natural sciences (e.g., physics, chemistry, biology, etc.). For example, researches are actively being conducted to design new materials or develop new drugs, and these researches are playing an important role in future technological advancement and industrial innovation.

However, a final stage in the development of all organic materials is to directly synthesize molecules, which requires related researchers to spend a lot of time and money performing chemical synthesis such as direct molecular synthesis.

Accordingly, researches are actively being conducted on methods for increasing the efficiency of natural science research based on generative artificial intelligence.

### [Disclosure]

### [Technical Problem]

The present invention provides an answer generation method and system capable of suggesting an optimal research method to researchers in the field of natural sciences.

More specifically, the present invention is to provide an answer generation method and system of a model capable of minimizing the risk of failure in natural science research based on a generative model to increase the efficiency of natural science research.

In addition, the present invention provides an answer generation method and system capable of solving time and cost problems required for material research and development and increasing the efficiency of material research and development.

### [Technical Solution]

An answer generation method performed by cooperation of a memory and at least one processor according to various embodiments disclosed in the present document may include: specifying an analysis target document; extracting a plurality of content from the document; storing the plurality of content extracted from the document in the memory; receiving a user query from a user terminal; specifying specific content related to the user query among the plurality of content stored in the memory; processing the specific content as input to a pre-trained chemical reaction prediction model; and generating an answer to the user query using output data of the chemical reaction prediction model.

In an embodiment, the answer generation method may further include: performing labeling so that a label is assigned to at least some of the plurality of content; and providing a graphic object corresponding to each content to which the label is assigned to a region of a service page where the user query is received.

In an embodiment, the answer generation method may further include: analyzing a relationship between the plurality of content based on a meaning of each of the plurality of content; and grouping related content among the plurality of content based on the relationship, in which, in the performing of the labeling, the same label is assigned to the grouped content through the grouping.

In an embodiment, in the extracting of the plurality of content, the plurality of content satisfying a preset content criterion may be extracted using a document understanding model.

In an embodiment, the preset content criterion may be related to contents related to a molecular structure related to at least one of chemistry, bio, new materials, new substances, and new drug development.

In an embodiment, in the document understanding model, at least one of a text, a molecular structure, a formula, a chart, a table, and an image satisfying the preset contents may be extracted from the document as the plurality of contents.

In an embodiment, in the grouping, contents for the same molecular structure among at least one of the text, molecular structure, the formula, the chart, the table, and the image extracted from the plurality of content may be grouped as the related content.

In an embodiment, the grouped content may include at least one of a molecular structure image, a name, a property, and a string according to a SMILES notation of a specific molecular structure corresponding to the grouped content.

In an embodiment, at least some of the content included in the grouped content for the specific molecular structure may be generated by at least one of the ultra-large foundation model, the pre-trained chemical reaction prediction model, and the pre-trained molecular property prediction model.

In an embodiment, in the specifying of the specific content, the user query may be analyzed to extract a label indicating the grouped content from the query, specific grouped content corresponding to the label may be specified, and a molecular structure of the specific grouped content may be processed as input to the prediction model, and in the generating of the answer, the answer may be generated using output data of the prediction model and contents constituting the grouped content.

In an embodiment, the generating of the answer to the user query may include: determining an answer generation procedure performed for prediction corresponding to the user query and a tool used in the answer generation procedure; providing information on the determined answer generation procedure and tool to the service page; and generating the answer to the user query using the determined answer generation procedure and tool.

In an embodiment, in the extracting of the plurality of content, contents related to a molecular structure related to at least one of chemistry, bio, new materials, new substances, and new drug development may be extracted from the document, and the content to which the label is assigned may be content related to the molecular structure extracted from the document, and the one region may include a graphic object corresponding to the extracted molecular structure.

In an embodiment, the one region may include a plurality of graphic objects each corresponding to a plurality of molecular structures when the plurality of molecular structures are extracted from the document, a first graphic object among the plurality of graphic objects may include an image of a first molecular structure corresponding to the first graphic object among the plurality of molecular structures, and a second graphic object among the plurality of graphic objects may include an image of a second molecular structure corresponding to the second graphic object among the plurality of molecular structures.

In an embodiment, the document may be provided to another region distinguished from the one region of the service page, and highlighting objects may overlap with a first region including the first molecular structure of the document provided to the service page and a second region including the second molecular structure, respectively, so that it is identified that the first molecular structure and the second molecular structure were extracted from the document.

In an embodiment, in the first region, a first label assigned to correspond to the first molecular structure may be provided around a first highlighting object overlapping with the first region, and in the second region, a second label assigned to correspond to the second molecular structure may be provided around a second highlighting object overlapping with the second region.

In an embodiment, the answer generation method may further include providing detailed information on a graphic object selected according to the user input to the service page based on receiving user input for selecting one of the plurality of graphic objects, in which the detailed information includes at least one of a molecular structure image of a specific molecular structure corresponding to the selected graphic object, a name of the molecular structure, a description of the molecular structure, a property of the molecular structure, and a SMILES notation of the molecular structure.

An answer generation system of ultra-large foundation model according to various embodiments disclosed in the present document may include: a memory and at least one processor, in which the memory and the processor cooperate to specify an analysis target document, extract a plurality of content from the document, receive a user query from a user terminal, and specify specific content related to the user query among the plurality of content, and the specific content is processed as input to a pre-trained prediction model, and generates an answer to the user query using output data of the prediction model.

According to another aspect of the present invention, a program stored on a computer-readable recording medium, executable by one or more processes on an electronic device may include instructions to execute: specifying an analysis target document; extract a plurality of content from the document; receiving a user query from a user terminal; specify specific content related to the user query among the plurality of content; processing the specific content as input to a pre-trained chemical reaction prediction model; and generating an answer to the user query using output data of the prediction model.

An answer generation method performed by cooperation of a memory and at least one process according to various embodiments disclosed in the present document may include: extracting at least one molecular structure from an analysis target document using a document understanding model; storing the molecular structure extracted from the document in the memory; performing labeling on the extracted molecular structure so that different labels rare assigned to each extracted molecular structure stored in the memory; receiving a user query including at least one of the labels assigned through the labeling through a service page; and generating an answer to the user query using a molecular structure corresponding to a specific label included in the user query among the extracted molecular structures. In an embodiment, the service page may include at least one of a first region in which information extracted from the document is provided, a second region in which at least a portion of the document is provided, and a third region in which the user query is received, the first region may include at least one graphic object corresponding to the extracted molecular structures to which the different labels are respectively assigned through the labeling, and at least one of detailed information on the extracted molecular structure, and the detailed information on the extracted molecular structures may include at least one of a molecular structure image, a name, a property, and a string according to the SMILES notation of the extracted molecular structure.

In an embodiment, the answer generation method may further include generating the detailed information on the extracted molecular structure, in which the detailed information may be extracted from the document or acquired from at least one pre-trained prediction model, the pre-trained prediction model may include at least one of a chemical reaction prediction model that predicts a chemical reaction between molecular structures and a molecular property prediction model that predicts a property of the molecular structure.

In an embodiment, the first region may include a first sub-region including the graphic object and a second sub-region including the detailed information, when the plurality of molecular structures are extracted from the document, the first sub-region may include a plurality of graphic objects corresponding to each of the plurality of molecular structures, and the detailed information on the molecular structure corresponding to one graphic object selected by a user input among the plurality of graphic objects may be provided in the second sub-region.

In an embodiment, the service page may be provided on an answer generation platform based on an ultra-large foundation model, and at least one of the analysis target document, the extracted molecular structure, the label for the extracted molecular structure, the user query, and the answer to the user query may be stored in a database (DB) of the platform by being linked to the user account.

In an embodiment, the generating of the answer may include processing a molecular structure corresponding to the specific label as input to the pre-trained prediction model, and generating the answer to the user query using output data of the pre-trained prediction model, and when the answer to the user query includes a specific molecular structure generated through the pre-trained prediction model, the label may be assigned to the specific molecular structure.

In an embodiment, the specific molecular structure and the label assigned to the specific molecular structure may be stored in a pre-specified storage together with the extracted molecular structure and the label assigned to the extracted molecular structure by being linked to a user account.

In an embodiment, the answer generation method may further include generating a specific graphic object corresponding to the specific molecular structure based on the specific molecular structure generated through the pre-trained prediction model and updating the first region so that the specific graphic object is included in the first region.

In an embodiment, in the generating of the answer to the user query, the property of the specific molecular structure may be predicted using the pre-trained prediction model, and as the answer to the user query, the information on the property of the predicted specific molecular structure may be provided together.

In an embodiment, based on the update, the information on the property of the specific molecular structure may be provided to the first region together with the specific graphic object.

In an embodiment, the answer generation method may include receiving a new user query including the label assigned to the specific molecular structure through the third region of the service page, and generating the answer to the new user query using at least a part of information on the specific molecular structure and the property of the specific molecular structure corresponding to the label assigned to the specific molecular structure in response to the new user query.

In an embodiment, the answer generation method may further include receiving an editing request for the extracted molecular structure through the service page to which the answer to the user query is provided and providing an editing interface that provides an editing function for the extracted molecular structure to the service page.

In an embodiment, the editing interface may include the molecular structure image of the extracted molecular structure, the molecular structure image may include nodes corresponding to each of the atoms constituting the extracted molecular structure and edges indicating a bond relationship of the atoms, the extracted molecular structure may be edited based on a user input for at least one of the nodes and the edges, and the edited molecular structure in which the extracted molecular structure is edited may be stored in a pre-specified storage.

In an embodiment, the edited molecular structure is assigned a new label specifying the edited molecular structure, and when the user query including the new label is input to the ultra-large foundation model, the ultra-large foundation model may generate an answer using the edited molecular structure corresponding to the new label.

In an embodiment, a graphic object corresponding to the edited molecular structure is provided to one region of the service page, and the graphic object corresponding to the edited molecular structure may include a molecular structure image of the edited molecular structure.

In an embodiment, the editing for the extracted molecular structure may be a deletion or position change of at least one of the nodes corresponding to each of the atoms constituting each of the extracted molecular structures and the edges indicating the bond relationship of the atoms, or an addition of a new node corresponding to a new atom or an addition of a new edge that generates a new bond relationship between the atoms.

An answer generation system of an ultra-large foundation model according to various embodiments disclosed in the present document may include: a memory and at least one processor, in which the memory and the processor cooperate to extract at least one molecular structure from an analysis target document using a document understanding model, perform labeling on the extracted molecular structure so that different labels are assigned to each extracted molecular structure, receive, through a service page, a user query including at least one of the labels assigned through the labeling, and generate an answer to the user query using a molecular structure corresponding to a specific label included in the user query among the extracted molecular structures.

A program according to various embodiments disclosed in the present document may include instructions to execute: extracting at least one molecular structure from an analysis target document using a document understanding model; performing labeling on the extracted molecular structure so that different labels are assigned to each extracted molecular structure; receiving, through a service page, a user query including at least one of the labels assigned through the labeling; and generating an answer to the user query using a molecular structure corresponding to a specific label included in the user query among the extracted molecular structures.

### [Advantageous Effects]

As described above, according to the answer generation method and system according to the present invention, by generating and providing the answer suitable for the user query based on the data extracted from the document, it is possible for the user to minimize the risk of research failure by receiving suggestions for the optimal research method.

In addition, according to the answer generation method and system according to the present invention, it is possible to provide the answer to the user query using the data that is extracted from the document or generated from the pre-trained prediction model. Accordingly, the user can quickly and accurately be provided with his/her required information and reduce the time and/or cost of research and/or development.

Furthermore, according to the answer generation method and system according to the present invention, it is possible to generate the answer to the user query using the predicted results from the pre-trained prediction model and provide the generated answer to the user. Accordingly, it is possible for the user to shorten the time required for research and/or development and reduce the number of trial and errors in research and/or development.

Furthermore, according to the answer generation method and system according to the present invention, by visualizing and providing the extracted molecular structure and related data through the user interface, it is possible for the user to intuitively recognize his/her required information and understand the information more quickly, thereby increasing the accuracy and efficiency of the research.

### [Description of Drawings]

FIG. 1 is a flowchart for describing an answer generation system according to the present invention.
FIGS. 2A and 2B and 3 are conceptual diagrams for describing an ultra-large foundation model according to the present invention.
FIG. 4 is a flowchart for describing an answer generation method according to the present invention.
FIGS. 5 to 27 are conceptual diagrams for describing the answer generation method according to the present invention.
FIGS. 28 to 30 are conceptual diagrams for describing a clustering method according to an embodiment of the present invention.
FIGS. 31 and 32 are conceptual diagrams for describing a method of generating a report according to an embodiment of the present invention.

### [Mode for Disclosure]

Hereinafter, embodiments disclosed in this specification will be described in detail with reference to the accompanying drawings, but the same or similar components will be denoted by the same reference numerals independent of the drawing numerals, and an overlapping description of the same or similar components will be omitted. In addition, the terms "module" and "unit" for components used in the following description are used only to easily make the disclosure. Therefore, these terms do not have meanings or roles that distinguish from each other in themselves. Further, in describing the embodiments disclosed in this specification, if it is determined that a detailed description of related known technologies may obscure the gist of the embodiments disclosed in this specification, the detailed description thereof is omitted. In addition, it is to be understood that the accompanying drawings are provided only for easy understanding of embodiments disclosed in this specification, and the technical idea disclosed in this specification is not limited by the accompanying drawings, but includes all the modifications, equivalents, and substitutions included in the spirit and the scope of the present invention.

The terms including ordinal numbers such as 'first' and 'second' may be used to describe various components, but these components are not limited by these terms. The terms are used to distinguish one component from another component.

It is to be understood that when one component is referred to as being "connected to" or "coupled to" another component, one component may be connected directly to or coupled directly to another component or be connected to or coupled to another component with the other component interposed therebetween. On the other hand, it is to be understood that when one component is referred to as being "connected directly to" or "coupled directly to" another component, it may be connected to or coupled to another component without the other component interposed therebetween.

Singular forms include plural forms unless the context clearly indicates otherwise.

It will be further understood that the terms "include" or "have" used in the present specification specify the presence of features, numerals, steps, operations, components, parts mentioned in the present specification, or combinations thereof, but do not preclude the presence or addition of one or more other features, numerals, steps, operations, components, parts, or combinations thereof.

The present invention relates to an answer generation method and system. The answer generation system according to the present invention performs answer generation based on generative artificial intelligence (generative AI) or a foundation model, and may also be called an answer generation platform based on an ultra-large foundation model. However, the "ultra-large foundation model" may also be called a generative model, a foundation model, or a large language model (LLM). The answer generation system according to the present invention may be a system that generates property prediction results of a molecular structure or designs a molecule having user's desired characteristics. In addition, the answer generation system according to the present invention may be a system that generates the predicted results of chemical reaction between a new type of molecules and/or a plurality of molecules. Furthermore, the answer generation system according to the present invention may be a system that generates predicted results of transformation of existing materials and synthesis of various materials (e.g., a new material, a polymer material, a nano material, a composite material, an organic material, a pharmaceutical material, etc.).

The answer generation system according to the present invention includes an ultra-large foundation model (or an ultra-large foundation artificial intelligence model), and the present invention aims to increase the efficiency of natural science research by minimizing the risk of research failure.

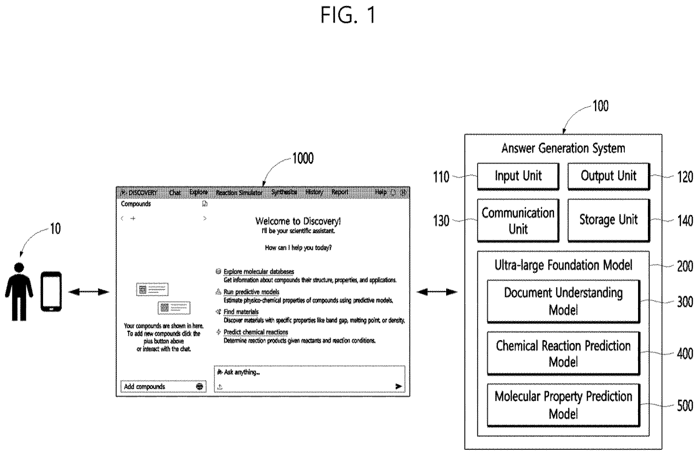

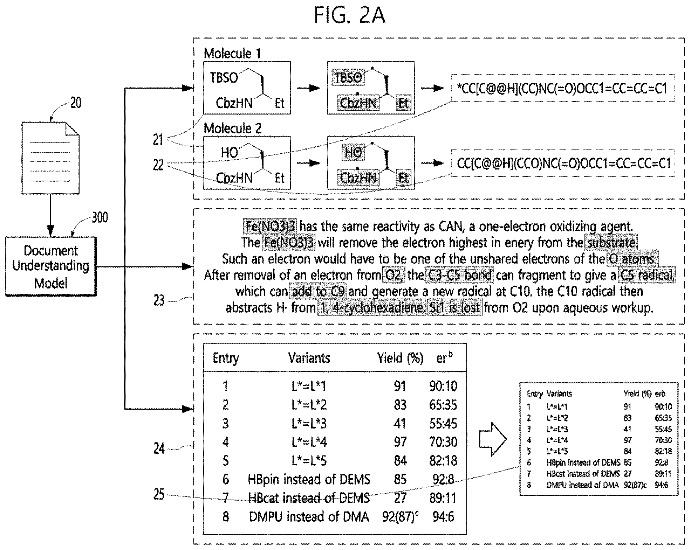

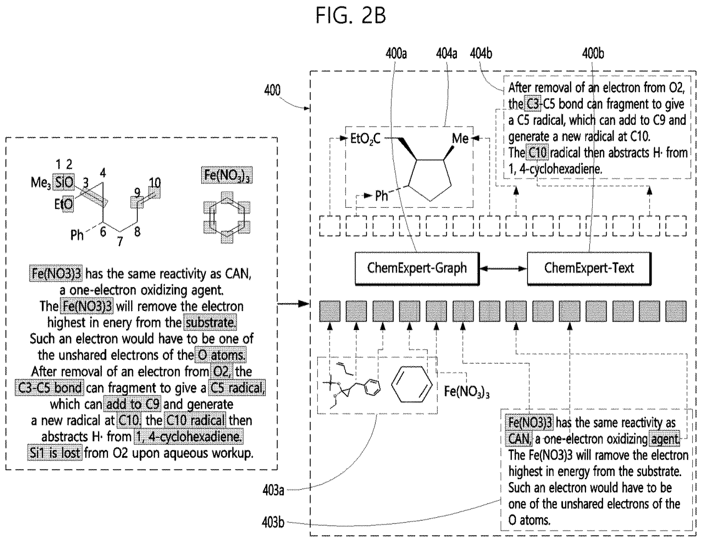

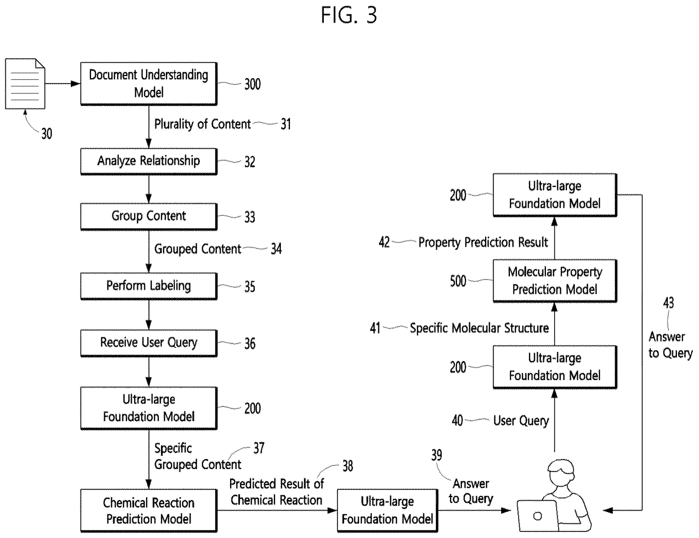

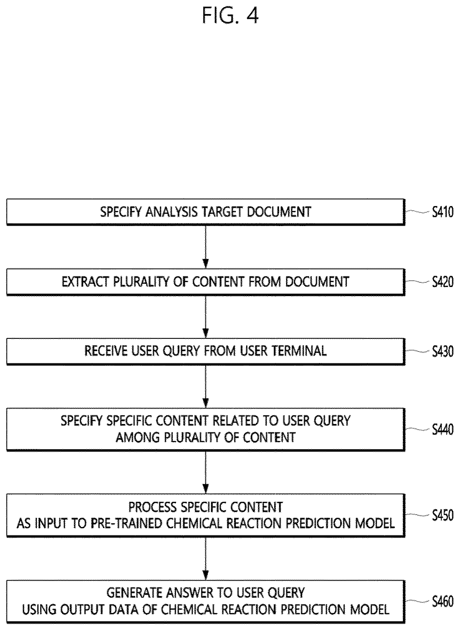

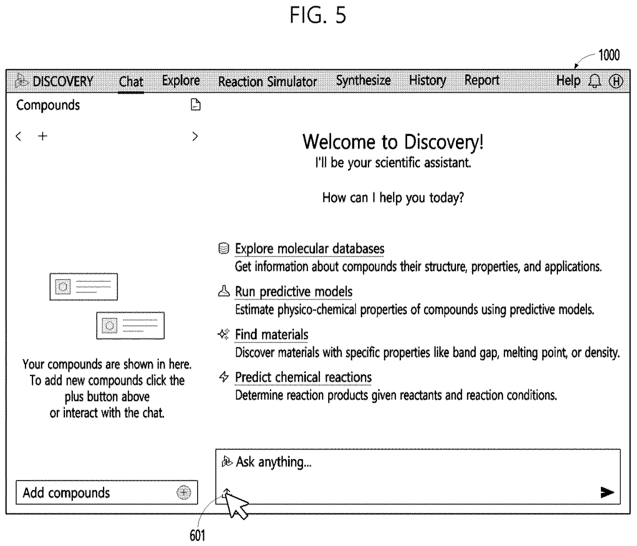

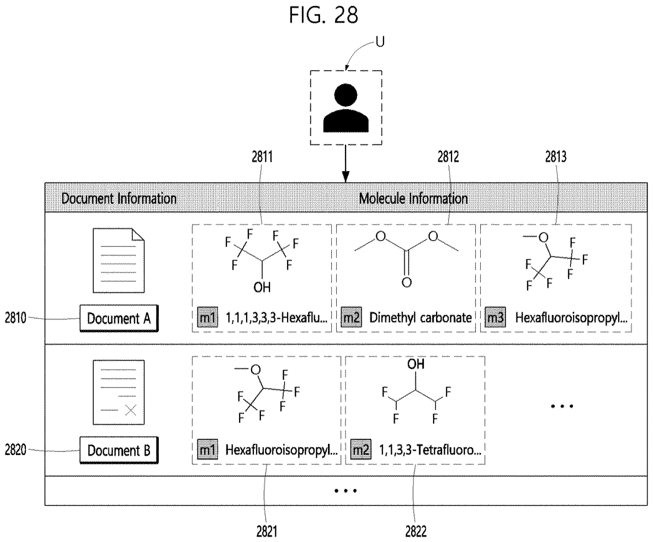

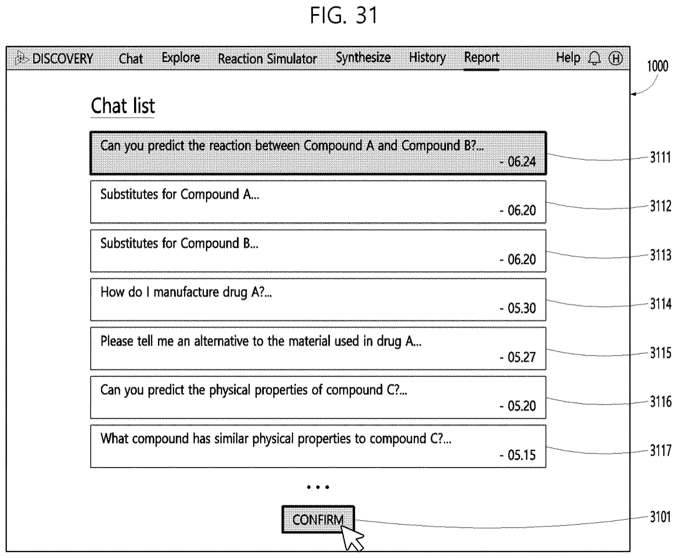

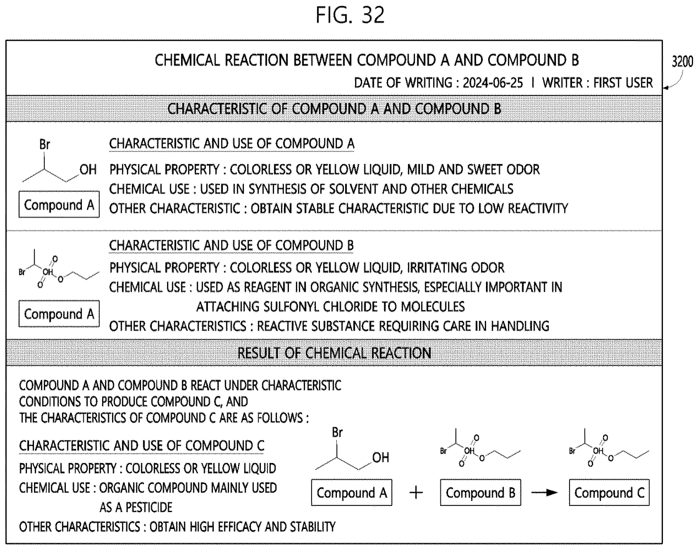

Hereinafter, it will be described in more detail with the attached drawings. FIG. 1 is a flowchart for describing an answer generation system according to the present invention, and FIGS. 2A and 2B and 3 are conceptual diagrams for describing an ultra-large foundation model according to the present invention. In addition, FIG. 4 is a flowchart for describing an answer generation method according to the present invention, and FIGS. 5 to 27 are conceptual diagrams for describing the answer generation method according to the present invention. Furthermore, FIGS. 28 to 30 are conceptual diagrams for describing a clustering method according to an embodiment of the present invention, and FIGS. 31 and 32 are conceptual diagrams for describing a method of generating a report according to an embodiment of the present invention.

Meanwhile, as illustrated in FIG. 1, an answer generation system 100 according to the present invention may include an input unit 110, an output unit 120, a communication unit 130, a storage unit 140, and an ultra-large foundation model 200.

Although not illustrated, the answer generation system 100 according to the present invention may include one or more processors, and the processors may include one or more general-purpose processors and/or one or more special-purpose processors (e.g., a digital signal processor, a tensor processing unit (TPU), a graphics processing unit (GPU), a neural network processing unit (NPU), an application-specific integrated circuit, an application-specific integrated circuit (ASIC), etc.). The one or more processors may be configured to execute instructions stored (or included) in the storage unit 140, computer-readable instructions, and/or other instructions described herein. The answer generation system and method according to the present invention may perform data processing described below by cooperation of a memory and at least one processor. The processor may perform a series of operations and data processing using data and information stored in the memory. In this case, the memory may be a configuration of the storage unit 140.

Meanwhile, the input unit 110 is a means for data input, and may be configured in various types. For example, the input unit 110 may be configured to receive user input. The input unit 110 may be configured to receive the user input from the user terminal 10. Here, the "receiving input" may mean receiving an input signal (or selection signal) corresponding to user input based on input performed by a user through the configuration of the input unit provided in the user terminal 10.

In addition, the input unit 110 in the present invention does not necessarily mean a hardware means, and may be understood as a passage for receiving input from a user.

The input unit 110 may also be referred to as a user interface module. The input unit 110 may include a touch screen, a computer mouse, a keyboard, a keypad, a touch pad, a trackball, a joystick, a voice recognition module, or other similar devices. However, the present invention does not limit the type of the input unit 110.

Here, the user input may include documents, texts, images (or videos), voices, etc. In this case, the answer generation system 100 may further include a module for converting voice into text.

Next, the output unit 120 may output information through the configuration of the output unit (e.g., a display unit, a touch screen, a speaker, etc.) provided in the user terminal 10 linked to the answer generation system 100 according to the present invention. For example, the output unit 120 may output a page (or a service page, 1000) linked to the answer generation system 100 according to the present invention to the display unit of the user terminal 10. In addition, the output unit 120 does not necessarily mean a hardware means, and may be understood as a passage for outputting results to the user.

Next, the communication unit 130 may be connected to the user terminal 10, a server (e.g., a central server, an external server, etc.), a device, and at least one network, etc., through a wireless or wired network, and may be configured to receive or transmit the overall data and information necessary for the operation of the answer generation system 100 according to the present invention.

Here, the mobile terminal 10 may include at least one of a mobile phone, a smart phone, a notebook computer, a laptop computer, a slate PC, a tablet PC, an ultrabook, a desktop computer, a digital broadcasting terminal, personal digital assistants (PDA), a portable multimedia player (PMP), navigation, a wearable device (e.g., a smartwatch, a smart glass, and a head mounted display (HMD)), and the like.

Furthermore, the communication unit 130 may support various communication methods according to the communication standards of the communicating device.

For example, the communication unit 130 may be configured to communicate with a communication target using at least one of wireless LAN (WLAN), Wireless-Fidelity (Wi-Fi), Wireless Fidelity (Wi-Fi) direct, digital living network alliance (DLAN), Wireless Broadband (WiBro), World Interoperability for Microwave Access (WiMAX), High Speed Downlink Packet Access (HSDPA), High Speed Uplink Packet Access (HSUPA), Long Term Evolution (LTE), Long Term Evolution-Advanced (LTE-A), 5th Generation (5G) Mobile Telecommunication, Bluetooth^{™} Radio Frequency Identification (RFID), Infrared Data Association; IrDA), Ultra-Wideband (UWB), ZigBee, Near Field Communication (NFC), Wi-Fi Direct, and Wireless Universal Serial Bus (USB) technologies.

Meanwhile, the storage unit 140 serves to store various data related to the present invention and may include one or more non-transitory computer-readable storage media that may be read and/or accessed by at least one of the one or more processors 140.

The one or more computer-readable storage media may include volatile and/or nonvolatile storage components such as optical, magnetic, organic or other memory or disk storage devices. In some examples, the storage unit 140 may be implemented using a single physical device (e.g., one optical, magnetic, organic, or other memory or disk storage device), while in other examples, the storage unit 140 may be implemented using two or more physical devices.

The storage unit 140 may include computer-readable instructions and additional data. The storage unit 140 may include storage necessary to perform at least some of methods, scenarios and techniques described herein and/or at least some of the functions of the devices and networks.

Furthermore, at least a portion of the storage unit 140 may be a cloud storage or a cloud server. The storage unit 140 may store at least some of data corresponding to the user input received from the input unit 110 and training data.

That is, the storage unit 140 may be sufficient as a space where information necessary for the operation of the answer generation system 100 according to the present invention is stored, and it may be understood that there is no limitation on the physical space.

Meanwhile, the ultra-large foundation model 200 may be configured to predict properties from a molecular structure or to design a molecule having user's desired characteristics. In addition, the ultra-large foundation model 200 may be configured to predict synthesis results between new types of molecules or between a plurality of molecules.

Here, an ultra-large foundation model 200 may also be referred to as a foundation model, and the foundation model may mean an ultra-large AI core foundation model trained with a massive dataset.

In this regard, the ultra-large foundation model 200 may include at least one of a document understanding model 300, a chemical reaction prediction model 400, and a molecular property prediction model 500.

The document understanding model 300 may extract various types of content that satisfy preset content criteria from documents (e.g., papers, books, patent documents, reports, etc.). More specifically, the document understanding model 300 may be a model trained to understand structured data, unstructured data, linguistic data (or linguistic elements), non-linguistic data (or non-linguistic elements), etc., included in a document, and extract various content (or data) and knowledge based on the understood contents.

Here, the preset content criteria may be set in various ways and may be determined according to the purpose or utilization purpose of the answer generation system 100 according to the present invention.

For example, when the utilization purpose of the answer generation system 100 is the chemistry, the bio, the new materials, the new substances, and the new drug development, the document understanding model 300 may be trained to understand and extract the contents related to the chemistry, the bio, the new materials, the new substances, and the new drug development from an analysis target document.

In this case, the preset content criteria may include the contents related to the molecular structures that are related to at least one of the chemistry, the bio, the new materials, the new substances, and the new drug development. Here, the document understanding model 300 may extract the contents related to the chemistry, the bio, the new materials, the new substances, and the new drug development from the analysis target document based on the preset content criteria.

In this specification, for the convenience of description, the preset content criteria are described as being related to at least one of the chemistry, the bio, the new materials, the new substances, and the new drug development, but are not necessarily limited thereto.

Based on the preset content criteria, the document understanding model 300 may extract at least one of the text, molecular structure, formula, chart, table, and image, which satisfy the preset content criteria, from the analysis target document.

In an embodiment, as illustrated in FIG. 2, the document understanding model 300 may understand the chemical structure of the molecular structure formula 21 included in the analysis target document 20, and may be extracted by converting a molecular structure formula 21 into a SMILES string expression formula 22 based on the result of understanding. In addition, the document understanding model 300 may understand the chemical structure of the molecular structure formula 21 and perform graph transformation corresponding to the molecular structure formula 21 based on the result of understanding.

In other embodiments, the document understanding model 300 may understand text 23 related to the molecular structure formula 21 among the texts included in the analysis target document 20, and extract the understood text 23 as text data 23 related to the molecular structure formula 21.

In another embodiment, the document understanding model 300 may recognize rows and columns that constitute a table 24 related to the molecular structure formula 21 from the analysis target document 20 and extract structured data 25 by converting the recognized rows and columns into the structured data 25 in a format such as HTML or Excel.

Furthermore, the document understanding model 300 may extract relationship information (or relationship) between the molecular structures included in the document.

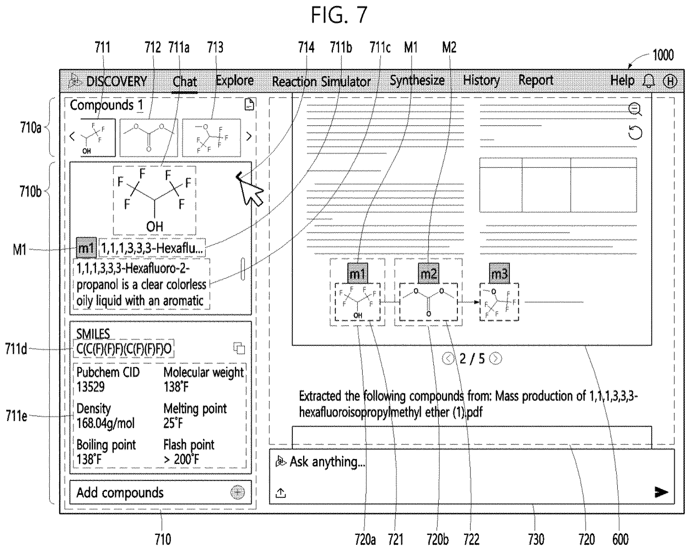

In an embodiment, as illustrated in FIG. 7, among the plurality of molecular structures included in an analysis target document 600, a third molecular structure to which a third label M3 is assigned may be understood as the molecular structure (or compound) generated as the results of the chemical reaction between the first molecular structure to which a first label M1 is assigned and the second molecular structure to which a second label M2 is assigned. The document understanding model 300 may understand the relationship between the first molecular structure and the second molecular structure included in the analysis target document 600, and extract the relationship information that the third molecular structure is generated through the chemical reaction between the first molecular structure and the second molecular structure.

In this case, the relationship information between the molecular structures may be extracted by understanding the text included in the analysis target document, or extracted by understanding non-verbal data included in the analysis target document.

In an embodiment, the document understanding model 300 may understand the relationship between the first molecular structure and the second molecular structure through symbols (e.g., plus, arrow, etc.) present in one region where the first molecular structure and the second molecular structure are located among the plurality of regions included in the analysis target document 600, and extract the relationship information that the third molecular structure is generated through the chemical reaction between the first molecular structure and the second molecular structure.

As described above, the document understanding model 300 may extract various types of data included in a document by converting the data into data (e.g., machine-readable data) in a form that the ultra-large foundation model 200 may understand. The data extracted using the document understanding model 300 may be sorted in units of pages or documents and stored in the storage unit 140 (or memory). In the present invention, the document understanding model 300 may also be called a deep document understanding model.

Next, the chemical reaction prediction model 400 may be a pre-trained model based on various training data to predict the results of the chemical reaction or the results of synthesis of various materials.

For example, the training data used for training the chemical reaction prediction model 400 may be data including structural information of reactants in various chemical reactions, information on reaction conditions, information on physical and/or chemical properties of products, results of chemical reactions observed in research (or experiments), etc.

Specifically, the chemical reaction prediction model 400 may predict the results of the chemical reaction between multiple molecules or new types of molecules, or predict the results of transformation of existing materials and synthesis of various materials (e.g., new materials, polymer materials, nanomaterials, composite materials, organic materials, pharmaceutical materials, etc.).

In addition, the chemical reaction prediction model 400 may predict the molecular structure of a substance (or compound or product) that may be generated under specific reaction conditions, analyze the reaction mechanism under specific conditions, and predict potential byproducts or side reactions to output optimal reaction conditions.

In an embodiment, the chemical reaction prediction model 400 may receive structural information of a specific compound and output the predicted results of the chemical reaction based on the input information and trained knowledge. In another embodiment, the chemical reaction prediction model 400 may receive specific chemical reaction condition and output the predicted reaction products based on the received information and trained knowledge.

In another embodiment, the chemical reaction prediction model 400 may receive structural information on a specific compound and output reaction condition information and reaction path information according to the received information and trained knowledge.

Meanwhile, as illustrated in FIG. 2B, the chemical reaction prediction model 400 may include at least one of a first module 400a and a second module 400b. Here, the first module 400a may also be named as a "ChemExpert-Graph" module, a graph module, a graph processing module, a graph model, a graph processing model, etc., and the second module 400b may also be called as a "ChemExpert-Text" module, a text module, a text processing module, a text model, a text processing model, etc.

The first module 400a may receive a molecular (or chemical) structure as input and predict a graph-based chemical reaction. For example, referring to (a) of FIG. 2D, an example of the chemical reaction may be confirmed. As illustrated in (b) of FIG. 2D, the first module 400a may include a plurality of layers 400a-1 to predict the graph-based chemical reaction. More specific details regarding the plurality of layers 400a-1 will be described later.

A molecular structure 403a (or molecular structure formula) input to the first module 400a is converted into a molecular graph in the form of a graph, and atoms in the molecular graph may be expressed as nodes and bonds may be expressed as edges.

The molecular structure 403a may correspond to at least one of data extracted from a document 403 including a molecular structure 411 using the document understanding model 300, or information extracted through the storage unit 140 (or memory).

The first module 400a may analyze changes in structural characteristics of a molecule based on the input molecular graph, predict a chemical reaction path and a product to be generated as the results of the chemical reaction, and output the predicted chemical reaction path and product.

In an embodiment, the first module 400a may analyze structural changes of a molecule based on the molecular graph, and predict a process in which a specific bond is separated and a new bond is formed.

In another embodiment, the first module 400a may analyze the interaction between the atoms in the molecule based on the molecular graph, and predict radical formation and bond changes that may occur at each step.

That is, the first module 400a may be configured to receive the molecular graph as input, and output the predicted chemical reaction path and a product 404a based on the molecular graph.

Next, the second module 400b may be configured to process text data 403b to understand and predict a reaction mechanism. In this case, the text data 403b may correspond to at least one of data extracted from a document including the molecular structure 403a using the document understanding model 300, or information extracted through the storage unit 140 (or memory) related to the molecular structure 411. The second module 400b may be a model that has pre-trained vast data related to the chemical reaction.

In an embodiment, the text data 403b input to the second module 400b is data including a description of the molecular structure 403a, and may include at least one of chemical reaction conditions, chemical reaction mechanisms (or reaction paths), and chemical characteristics of the molecular structure 403a.

The second module 400b may analyze the input text data 403b to understand and predict the chemical reaction mechanisms. More specifically, the second module 400b may analyze the input text data 403b and output at least one of the chemical reaction conditions, chemical reaction mechanisms, and chemical characteristics that are predicted based on the text data 403b.

In an embodiment, the second module 400b may analyze the text data 403b using a natural language processing (NLP) technology and extract at least one text of the chemical reaction conditions, chemical reaction mechanisms (or reaction paths), chemical characteristics, and experimental data included in the text data 403b.

In another embodiment, the second module 400b may predict the chemical reaction mechanisms (e.g., how a specific catalyst or condition affects the reaction) based on the text extracted through the analysis of the text data 403b, and output the predicted chemical reaction mechanisms and chemical characteristics.

The second module 400b may analyze the information related to the chemical reaction prediction, which is related to the plurality of molecular structures, from the text data.

The chemical reaction prediction model 400 may combine the output data 404a of the first module 400a and the output data 404b of the second module 400b to output the predicted results (e.g., product, chemical reaction path, chemical reaction mechanism, etc.) of a final chemical reaction.

In an embodiment, as illustrated in (a) to (c) of FIG. 2C, the chemical reaction prediction model 400 may generate electron flow, reaction conditions, and structural effects of a molecular structure (or chemical structure) using the output data output from the first module 400a and the second module 400b. In this case, the electron flow, the reaction conditions, and the structural effects may be expressed together as the graph and text, the molecular structure reflecting the position before and after the electron moves may be generated, or the molecular structure of the product generated according to the reaction conditions may be generated.

That is, the chemical reaction prediction model 400 can make more accurate predictions than using only a single data source by fusing the output data 404a and 404b output from the first module 400a and the second module 400b, respectively, and may enable users to intuitively recognize various elements related to chemical reactions.

In addition, the chemical reaction prediction model 400 may verify the chemical reaction products predicted through the first module 400a using the output data analyzed in the second module 400b. That is, the second module 400b may acquire at least one of the chemical reaction conditions, chemical reaction mechanisms, and chemical characteristics analyzed based on the text data 403b. The chemical reaction prediction model 400 may verify whether the chemical reaction products predicted and acquired in the first module 400a match the experimental data or theoretical expectations based on the data analyzed in the second module 400b.

Next, a molecular property prediction model 500 may be a model pre-trained based on various training data to predict properties of a substance (or molecule) or design a material structure.

For example, the training data used for training the molecular property prediction model 500 may be data including unique characteristic information of the substance and property information of the substance.

Here, the unique characteristic information of the substance may include the name of the substance, the molecular structural formula, and/or chemical the formula, etc. In addition, the property information of the substance may include property (i.e., domain) values such as boiling point, melting point, refractive index, solubility, viscosity, surface tension, density, strength, and/or thermal conductivity of the substance.

The molecular property prediction model 500 may predict properties (or property information) of a substance or design a material having user's desired properties.

Specifically, the molecular property prediction model 500 may receive the unique characteristic information of the substance and/or the property information of the substance, and output predicted data based on the input information and trained knowledge.

In an embodiment, the molecular property prediction model 500 may receive unique characteristic information of a specific substance, and output property information of the specific substance predicted based on the input information and trained knowledge.

In another embodiment, the molecular property prediction model 500 may receive property information of a specific substance, and output unique characteristic information of the specific substance predicted based on the input information and trained knowledge.

In another embodiment, the molecular property prediction model 500 may receive the unique characteristic information of the specific substance and the property information of the specific substance, and output optimal unique characteristic information of the substance and property information of the substance predicted based on the input information and trained knowledge.

As described above, the answer generation system 100 based on the ultra-large foundation model 200 is intended to suggest optimal research methods to researchers in the field of natural science, minimize the risk of failure in natural science research, and increase the efficiency of natural science research. More specifically, the present invention is to solve the problem of time and cost required for material research and development and to increase the efficiency of material research and development. Hereinafter, the answer generation method of the ultra-large foundation model and the overall process of the system will be described.

The answer generation system 100 may specify an analysis target based on the user input received from the user terminal 10. Here, the user input may include at least one of a document, an image, a voice, a video, and a text. For example, when the user input for the document is received, the answer generation system 100 may specify the document corresponding to the user input as an analysis target. Hereinafter, it will be described on the premise of the process of receiving the user input for the document.

As illustrated in FIG. 3, the answer generation system 100 may specify an analysis target document 30 to be analyzed.

In the present invention, there may be various methods (or methods or criteria) for specifying the analysis target document 30.

In an embodiment, the answer generation system 100 may specify the input document as the analysis target document 30 based on the fact that at least one document corresponding to the user selection among documents stored (or embedded) in the storage (or memory or storage space or database) of the user terminal 10 is input to a document upload page (or interface) provided to a service page 1000.

In another embodiment, the answer generation system 100 may receive link information (e.g., URL) of a document or link information of external storage services (e.g., Google Drive, Dropbox, etc.) storing the document from the user terminal 10. The answer generation system 100 may directly access the document through the link information of the document or download the document to specify the analysis target document 30.

However, the method of specifying an analysis target document in the present invention is not necessarily limited to the above-described embodiment. Hereinafter, for the convenience of description, it will be described on the premise that the document received through the user terminal 10, to which the service page 1000 is output, is specified as the analysis target document 30.

When the analysis target document 30 is specified, the answer generation system 100 may extract various forms of content from the analysis target document 30 using the document understanding model 300. Here, various types of content extracted from the analysis target document 30 may be understood as content satisfying the preset content criteria.

As described above, based on the fact that the purpose of using the answer generation system 100 is the chemistry, the bio, the new materials, the new substances, and the new drug development, the preset content criteria may be determined as contents related to at least one of the chemistry, the bio, the new materials, the new substances, and the new drug development.

Here, the document understanding model 300 may extract the plurality of content 31 related to the chemistry, the bio, the new materials, the new substances, and the new drug development from the analysis target document 30 based on the preset content criteria. For example, the plurality of content 31 may include at least one of text, molecular structure, formula, chart, table, and image.

Furthermore, when the plurality of content 31 is extracted from the document understanding model 300, the processor may store the plurality of extracted content 31 in the storage unit 140 (or memory).

Meanwhile, the answer generation system 100 (or processor) may analyze the relationship between the plurality of content 31 stored in the storage unit 140 (or memory). Here, the relationship indicates the semantic association between different types of content (e.g., molecular structure, text, formula, table, etc.), and may mean the relationship based on semantic, thematic, and/or structural similarity that is connected between the different types of content. This relationship may be analyzed based on the meanings of each content.

Specifically, the answer generation system 100 may analyze (32) the relationship between the plurality of content 31 based on the meanings of each of the plurality of content 31. For example, it is assumed that the first molecular structure, the second molecular structure, a first text, a second text, a first formula, a second formula, a first table, and a second table are extracted as the plurality of content 31. The answer generation system 100 may specify that the first molecular structure, the first text, the first formula, and the first table have a mutual relationship through a relationship analysis 32 of the plurality of content 31, and may specify that the second molecular structure, the second text, the second formula, and the second table have the mutual relationship.

Furthermore, the answer generation system 100 may perform grouping of the plurality of content 31. In the present invention, the grouping may be performed between the plurality of content 31 that has the mutual relationship.

The answer generation system 100 may group 33 related content among the plurality of content 31 based on the relationship between the plurality of content.

More specifically, the answer generation system 100 may group 33 contents related to the same molecular structure among at least one text, molecular structure, formula, chart, table, and image included in the plurality of content 31 into related content to generate grouped content 34.

In an embodiment, the answer generation system 100 may group 33 the first text, the first formula, and the first table including contents related to the first molecular structure among the plurality of content 31 into the content related to the first molecular structure to generate the grouped first content.

In other embodiments, the answer generation system 100 may group the second text, the second formula, and the second table including the contents related to the second molecular structure among the plurality of content 31 into the content related to the second molecular structure to generate the grouped second content.

Furthermore, the answer generation system 100 (or processor) may store the grouped content in the storage unit 140 (or memory) by linking the grouped content to a user account.

Through the process described above, the content 34 grouped based on a specific molecular structure may include at least one of a molecular structure image of a specific molecular structure corresponding to the grouped content 34, a name of the molecular structure, a description of the molecular structure, properties (e.g., molecular weight, density, melting point, boiling point, flash point, surface tension, etc.) of the molecular structure, and a string according to the SMILES notation of the molecular structure.

Meanwhile, at least some of the content included in the grouped content 34 for the specific molecular structure may include content generated by the pre-trained prediction model.

Specifically, at least some of the content included in the grouped content 34 for the specific molecular structure may include the content generated by at least one of the ultra-large foundation model 200, pre-trained chemical reaction prediction model 400, and pre-trained molecular property prediction model 500.

In an embodiment, it is assumed that the plurality of content 31 extracted from the analysis target document 30 includes the molecular structure image and name of the specific molecular structure, and no description of the specific molecular structure exists. The answer generation system 100 may generate a description of the specific molecular structure using the pre-trained chemical reaction prediction model 400. In addition, the answer generation system 100 may group 33 the molecular structure image and name of the specific molecular structure extracted from the analysis target document 30 and the description of the specific molecular structure generated by the chemical reaction prediction model 400 to generate the grouped content 34.

In another embodiment, it is assumed that the plurality of content 31 extracted from the analysis target document 30 includes the molecular structure image, the name, and the description of the specific molecular structure, and no property of the specific molecular structure exists. The answer generation system 100 may generate the properties of the specific molecular structure using the pre-trained molecular property prediction model 500. In addition, the answer generation system 100 may group 33 the molecular structure image, name, and description of the specific molecular structure extracted from the analysis target document 30 and the properties of the specific molecular structure generated by the molecular property prediction model 500 to generate the grouped content 34.

That is, the answer generation system 100 may generate the content not included in the analysis target document using at least one of the ultra-large foundation model 200, chemical reaction prediction model 400, and molecular property prediction model 500, and generate the grouped content 34 including the content generated by at least one of the models 200, 400, and 500.

Meanwhile, the answer generation system 100 may perform labeling 35 so that labels are assigned to at least some of the plurality of content 31. Here, at least some of the content to which the label is assigned among the plurality of content 31 may correspond to the content 34 grouped through the grouping 33.

In this case, the grouped content 34 including the related content may be assigned the same label.

Specifically, the answer generation system 100 may assign the same label to the grouped content 34 through the labeling 35. For example, the answer generation system 100 may assign a first label to the first content grouped based on the first molecular structure, and assign a second label to the second content grouped based on the second molecular structure through the labeling 35.

In this regard, as described above, when there are the plurality of grouped content 34 in the present invention, each of the plurality of grouped content 34 may be assigned different labels (e.g., the first label may be assigned to the first grouped content, and the second label may be assigned to the second grouped content).

However, as discussed above, the present invention has described the labeled target to which the label is assigned as the grouped content, but it is not necessarily limited thereto. In the present invention, in addition to the grouped content, it is also possible to assign labels to each content by labeling each content having an independent meaning.

Furthermore, the grouped content to which different labels are assigned may be stored in the storage unit 140 in connection with the user account.

Meanwhile, the answer generation system 100 may provide the grouped content 34 stored in the storage unit 140 to the user terminal 10 to which the service page 1000 is output.

Specifically, the answer generation system 100 may provide a graphic object corresponding to each grouped content 34 assigned the label through the performance of the labeling 35 to a region of the service page 1000 on which the user query is received (e.g., see FIG. 7).

The answer generation system 100 may receive the user query corresponding to the user input through one region of the service page 1000.

Here, the user query 36 may include a label (or label information) assigned to the grouped content 34, or information (e.g., a name of the molecular structure, a chemical formula of the molecular structure, etc.) that may express the molecular structure.

Hereinafter, for the convenience of description, it is assumed that a user query 36 including label information (e.g., "Can you predict the chemical reaction of the first label M1 and the second label M2?") is received. However, the present invention does not limit the information included in the user query 36, and any information that may express the specific molecule (or compound or material) may be included in the user query.

Based on receiving the user query 36, the answer generation system 100 may process the user query 36 as the input of the ultra-large foundation model 200.

The ultra-large foundation model 200 may receive the user query 36 as the input, understand the contents included in the user query 36, and specify the specific content related to the user query 36.

More specifically, the ultra-large foundation model 200 may extract the label assigned to the grouped content 34 from the user query 36 through the analysis of the user query 36, and specify specific grouped content 37 corresponding to the extracted label. For example, based on the analysis result of the user query 36, which includes contents corresponding to the first label and the second label indicating the specific grouped content 37 in the user query 36, the ultra-large foundation model 200 may specify the first content corresponding to the first label and the second content corresponding to the second label as the specific grouped content 37 related to the user query 36.

Furthermore, the ultra-large foundation model 200 may process specific content (i.e., the molecular structure of the specific grouped content) as the input of the pre-trained chemical reaction prediction model 400.

In this case, the ultra-large foundation model 200 may change the name of the molecular structure included in the specific grouped content 37 to a language that the computer may understand.

More specifically, the ultra-large foundation model 200 may convert (or change) the name of the molecular structures included in the specific grouped content 37 into the string according to the SMILES notation which is the language that the computer may understand, and process the converted string and the information on the specific grouped content 37 as the inputs of the chemical reaction prediction model 400 that understands the chemical reaction mechanisms. For example, the ultra-large foundation model 200 may convert the names of the first molecular structure and the second molecular structure included in each of the specific grouped content 37 into the string according to the SMILES notation, and input the converted string and the information on the first molecular structure and the second molecular structure to the pre-trained chemical reaction prediction model 400.

The chemical reaction prediction model 400 may predict the intermolecular chemical reaction of the specific grouped content 37 and output the predicted results as the output data. As described above, the chemical reaction prediction model 400, which receives the string converted from the ultra-large foundation model 200 and the information on the first molecular structure and the second molecular structure, may predict the chemical reaction (or synthesis result) between the first molecule corresponding to the first molecular structure and the second molecule corresponding to the second molecular structure, and output a predicted result 38 of the chemical reaction between the first molecule and the second molecule.

In an embodiment, the predicted results of the chemical reaction may include a third molecular structure generated as the results of the chemical reaction between the first molecular structure and the second molecular structure, and may include at least one of the chemical characteristics, reaction conditions, expected yield, reaction energy, reaction path, and expected reaction time of the third molecular structure.

Meanwhile, the ultra-large foundation model 200 may generate an answer 39 to the user query 36 using the output data (predicted result 38 of chemical reaction) of the chemical reaction prediction model 400 and the content (or specific grouped content) constituting the grouped content.

In this case, the ultra-large foundation model 200 may determine what procedure and tool to use to generate the answer 39 to the user query 36. More specifically, the ultra-large foundation model 200 may determine the answer generation procedure performed for prediction corresponding to the user query 36 and the tool used for the answer generation procedure.

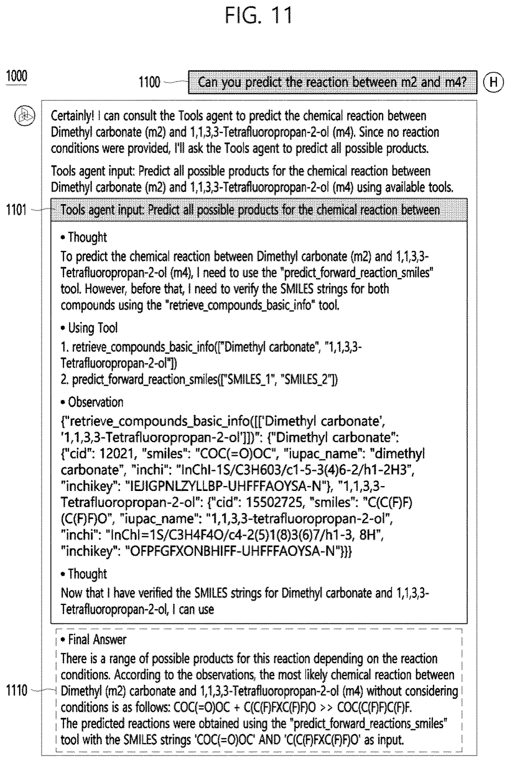

In this case, the answer generation system 100 may provide the information on the answer generation procedure determined from the ultra-large foundation model 200 and the tool used for the answer generation procedure to the service page 1000 (e.g., see FIG. 11).

The ultra-large foundation model 200 may execute an operation of generating the answer 39 to the user query 36 based on the determined answer generation procedure and tool. The ultra-large foundation model 200 may generate the answer 39 to the user query 36 using the output data of the chemical reaction prediction model 400 described above, the contents that constitutes the specific grouped content 37, and the determined answer generation procedure and tool.

In this case, the answer generation system 100 may assign a new label (e.g., the third label M3) through labeling the new molecular structure based on the fact that the answer 39 generated from the ultra-large foundation model 200 includes a new molecular structure (e.g., the third molecular structure).

Based on the generation of the answer 39 to the user query 36, the answer generation system 100 may provide the answer 39 generated from the ultra-large foundation model 200 through the user terminal 10 to which the service page 1000 is output.

Meanwhile, the answer generation system 100 may receive a new (or additional) user query through the service page 1000.

The answer generation system 100 may receive an input for a new user query 40 when the new user query 40 is input from the user terminal 10 after the answer 39 to the user query 36 is provided.

For example, the new user query 40 may be a query including contents related to at least one molecular structure to which a label is assigned, or a query including contents related to another molecule to which a label is not assigned. In the following, for the convenience of description, it will be described on the premise that the new user query 40 including a specific molecular structure 41 (e.g., the third molecular structure) to which a new label (e.g., the third label M3) is assigned is received.

The answer generation system 100 may input the new user query 40 to the ultra-large foundation model 200 based on receiving the new user query 40 including the label M3 assigned to the third molecular structure 41.

The ultra-large foundation model 200 may utilize at least one prediction model to understand the new user query 40 and generate an answer to the new user query 40. In an embodiment, the ultra-large foundation model 200 may input information on the third molecular structure 41 to the molecular property prediction model 500 based on the fact that the user query 40 includes the contents "Can you predict surface tension of m3?"

The molecular property prediction model 500 may predict the properties (e.g., surface tension) for the third molecular structure 41 corresponding to the new user query 40. In addition, the molecular property prediction model 500 may output a property prediction result 42 of the third molecular structure 41.

In an embodiment, the property prediction result 42 may include at least one of surface tension, boiling point and melting point, density, solubility, viscosity, thermal characteristics, mechanical characteristics, and electrical characteristics for the third molecular structure 41.

The ultra-large foundation model 200 may determine the answer generation procedure performed for prediction corresponding to the new user query 40 and the tool used in the answer generation procedure, and may generate an answer 43 (e.g., "m3 surface tension is OO...,) to the user query 40 using the determined answer generation procedure and tool and the output data of the molecular property prediction model 500.

In addition, the answer generation system 100 may provide the answer 43 generated from the ultra-large foundation model 200 to the user terminal 10.

In this way, the present invention may generate an answer suitable for a user query and generate the answer, thereby allowing the user to receive an optimal research method and minimize the risk of research failure. In addition, by providing the prediction information for the user query using the pre-trained prediction model, the user's decision-making may be supported, and the user may receive the information that the user needs, thereby increasing the efficiency of research.

Hereinafter, based on the answer generation method of the ultra-large foundation model and the overall process of the system described above, the answer generation method of the ultra-large foundation model will be described in more detail.

First, in the present invention, a process of specifying an analysis target document may be performed (S410, see FIG. 4).

The answer generation system 100 according to the present invention may be implemented in various platform forms such as applications, software, and websites. In this specification, for the convenience of description, the form in which the answer generation system 100 is implemented is not limited. In the present invention, the answer generation system 100 may also be called an "answer generation platform."

As described above, the user may have a user account pre-registered in the answer generation system 100 according to the present invention. In this case, the account may be generated through a page (or screen) linked to the answer generation system 100. Alternatively, the account can also be generated in at least one other system linked to the answer generation system 100. However, in this specification, the system to which the user account is issued is not separately distinguished, and all accounts that may use various services (or functions) provided by the answer generation system 100 according to the present invention are called "accounts pre-registered in the answer generation system 100."

Meanwhile, in the present invention, receiving the "molecular structure" may be understood as receiving information that may specify a molecule. In this case, the information that may specify the molecular structure may be in various forms such as the molecular structure formula, a molecular graph, the chemical formula, the molecular structure formula based on the SMILES notation, the molecular structure image, etc.

As illustrated in FIG. 5, the answer generation platform based on the ultra-large foundation model 200 may provide the service page 1000 linked to the platform to the user terminal 10.

The answer generation system 100 may receive the user input for at least one document from the user terminal 10 to which the service page 1000 is provided. In this case, the answer generation system 100 may receive one or more documents (i.e., a plurality of documents are also possible) from the user terminal 10, and in this specification, for the convenience of description, it will be described on the premise that one document is received.

In order to receive the user input for the document, the answer generation system 100 may provide (or display) a graphic object 601 linked to a document input function in one region of the service page 1000. For example, when the graphic object 601 is selected from the user terminal 10, the answer generation system 100 may activate (or output) a document upload page (or window) on the user terminal 10. The user may select a document through the document upload page or upload a document in a drag and drop manner. The answer generation system 100 may receive the user input for the document based on the input of the document corresponding to the user selection.

However, the user input for the document in the present invention is not necessarily limited to the above-described embodiments. As an example, a user may input link information (e.g., URL) of a document, or input link information of an external storage service (e.g., Google Drive, Dropbox, etc.) where the document is stored. In this case, the answer generation system 100 may directly access the document through the link information, or download the document, and receive the user input for the document.

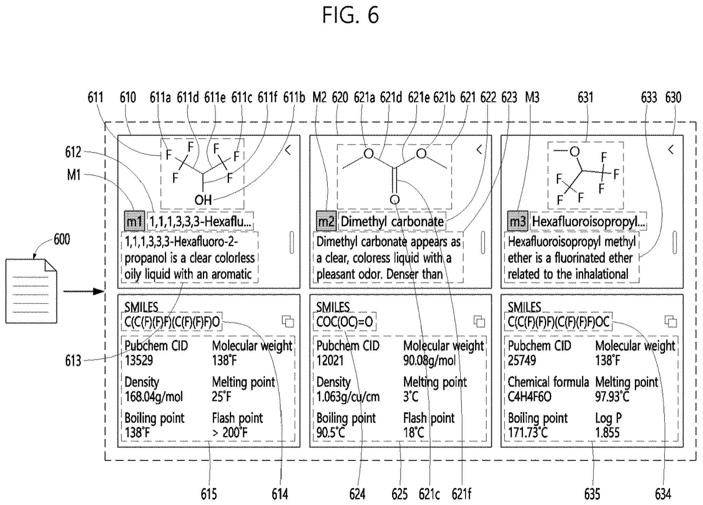

Furthermore, the answer generation system 100 may specify a document received from the user terminal 10 as an analysis target document. For example, as illustrated in FIG. 6, the answer generation system 100 may specify the document 600 corresponding to the user input as a target for analysis using the document understanding model 300.

When the analysis target document is specified, the present invention may proceed with a process (S420) of extracting the plurality of content from the analysis target document (see FIG. 4).

As described above, the document understanding model 300 may extract the plurality of content satisfying the preset content criteria from at least one document. Here, the preset content criteria may be understood as contents related to the molecular structure related to at least one of the chemistry, the bio, the new materials, the new substances, and the new drug development. Accordingly, the answer generation system 100 may extract contents related to the molecular structure related to at least one of the chemistry, the bio, the new materials, the new substances, and the new drug development from the document using the document understanding model 300.

Specifically, the answer generation system 100 may extract at least one molecular structure from the analysis target document 600 using the document understanding model 300. For example, as illustrated in FIG. 6, the document understanding model 300 may extract content corresponding to a first molecular structure 611, a second molecular structure 621, and a third molecular structure 631 from the analysis target document 600.

In addition, the answer generation system 100 may extract at least one of the text, the formula, the chart, the table, and the image as the plurality of content from the analysis target document 600 using the document understanding model 300. For example, the document understanding model 300 may extract texts 612, 613, 614, 615, 622, 623, 624, 625, 632, 633, 634, and 635 included in the analysis target document 600.

The plurality of content extracted from the document understanding model 300 may be stored in the storage unit 140 (or memory). For example, the answer generation system 100 may store the plurality of extracted molecular structures 611, 621, and 631 and the extracted texts 612, 613, 614, 615, 622, 623, 624, 625, 632, 633, 634, and 635 together with the plurality of extracted molecular structures 611, 621 and 631 in the storage unit 140.

Meanwhile, the answer generation system 100 may analyze the relationship between the plurality of content based on the meanings of each of the plurality of content stored in the storage unit 140. That is, the answer generation system 100 or the processor of the system 100 may perform a series of processes described in the present invention using the content in the storage unit 140 (or memory).

Here, the relationship analysis can be understood as a process of identifying and understanding the mutual relationship (or relevance) between the plurality of content based on the meanings of each of the plurality of content. This may include a process of identifying similarity, mutual dependency, or linked meaning of the information expressed by each content and grouping the similarity, mutual dependency, or linked meaning, or deriving a specific pattern.

The answer generation system 100 may analyze the meanings of each of the plurality of content and specify (or extract) the meanings of each of the plurality of content. In this case, the analysis of the meanings of each of the plurality of content may also be performed by at least one of the ultra-large foundation model 200, the chemical reaction prediction model 400, and the molecular property prediction model 500. For example, the answer generation system 100 may analyze the meaning of each of the texts 612, 613, 614, 615, 622, 623, 624, 625, 632, 633, 634, and 635 extracted from the document understanding model 300, and based on the analysis results, determine that the meaning of each of the texts 612, 613, 614, 615, 622, 623, 624, 625, 632, 633, 634, and 635 has the name 612 of the first molecular structure, the description 613 of the first molecular structure, the SMILES notation 614 of the first molecular structure, the property 615 of the first molecular structure, the name 622 of the second molecular structure, the description 623 of the second molecular structure, the SMILES notation 624 of the second molecular structure, the property 625 of the second molecular structure 625, the name 632 of the third molecular structure, the description 633 of the third molecular structure, the SMILES notation 634 of the third molecular structure, and the property 635 of the third molecular structure.

Furthermore, the answer generation system 100 may specify the content having the mutual relationship based on the meanings of each of the plurality of specified content. More specifically, the answer generation system 100 may specify the relationship between the plurality of molecular structures 611, 621 and 631 extracted from the document understanding model 300, the name 612 of the first molecular structure with different meanings, the description 613 of the first molecular structure, the SMILES notation 614 of the first molecular structure, the property 615 of the first molecular structure, the name 622 of the second molecular structure, the description 623 of the second molecular structure, the SMILES notation 624 of the second molecular structure, the property 625 of the second molecular structure, the name 632 of the third molecular structure, the description 633 of the third molecular structure, the SMILES notation 634 of the third molecular structure, and the property 635 of the third molecular structure. For example, the answer generation system 100 may specify that there are the mutual relationships between the first molecular structure 611 and the name 612 of the first molecular structure, the description 613 of the first molecular structure, the SMILES notation 614 of the first molecular structure, and the property 615 of the first molecular structure, and that there are the mutual relationships between the second molecular structure 621 and the name 622 of the second molecular structure, the description 623 of the second molecular structure, the SMILES notation 624 of the second molecular structure, and the property 625 of the second molecular structure. In addition, the answer generation system 100 may specify that there is the mutual relationship between the third molecular structure 631, the name 632 of the third molecular structure, the description 633 of the third molecular structure, the SMILES notation 634 of the third molecular structure, and the property 635 of the third molecular structure.

Meanwhile, the answer generation system 100 may group contents about the same molecular structure among the plurality of content into content related to each other based on the relationship between the plurality of content. More specifically, the answer generation system 100 may group, based on the specific relationship, the name 612 of the first molecular structure, the description 613 of the first molecular structure, the SMILES notation 614 of the first molecular structure, and the property 615 of the first molecular structure, which include the contents related to the first molecular structure 611, into the content related to the first molecular structure 611. In addition, the answer generation system 100 may group, based on the specified relationship, the name 622 of the second molecular structure, the description 623 of the second molecular structure, the SMILES notation 624 of the second molecular structure, and the property 625 of the second molecular structure, and the property 625 of the second molecular structure, which include the contents related to the second molecular structure 621, into the content related to the second molecular structure 621. Furthermore, the answer generation system 100 may group, based on the specific relationship, the name 632 of the third molecular structure, the description 633 of the third molecular structure, and the SMILES notation 634 of the third molecular structure, and the property 635 of the third molecular structure, which include the contents related to the third molecular structure 631, into the contents related to the third molecular structure 631.

Through the grouping process described above, at least one grouped content may be generated (or extracted). Referring to FIG. 6, the first molecular structure 611, the name 612 of the first molecular structure, the description 613 of the first molecular structure, the SMILES notation 614 of the first molecular structure, and the property 615 of the first molecular structure may be grouped to generate the grouped first content 610. In addition, the second molecular structure 621, the name 622 of the second molecular structure, the description 623 of the second molecular structure, the SMILES notation 624 of the second molecular structure, and the property 625 of the second molecular structure may be grouped to generate the grouped second content 620. Furthermore, the third molecular structure 631, the name 632 of the third molecular structure, the description 633 of the third molecular structure, the SMILES notation 634 of the third molecular structure, and the property 635 of the third molecular structure may be grouped to generate the grouped third content 630.

The content 610, 620 and, 630 grouped based on each of the plurality of molecular structures 611, 621 and 630 may include at least one of the molecular structure images of the specific molecular structures corresponding to each of the grouped content 610, 620 and, 630, the names 612, 622, and 632 of the molecular structure, the descriptions 613, 623, and 633 of the molecular structure, the strings 614, 624, and 634 according to the SMILES notation of the molecular structure, and the properties 615, 625, and 635 of the molecular structure.

Furthermore, the grouped content 610, 620 and, 630 may be stored in the storage unit 140 by being linked to the user account.

Meanwhile, the answer generation system 100 may perform the labeling on each of the grouped content 610, 620 and, 630 stored in the storage unit 140 so that the labels are assigned to each of the grouped content 610, 620 and, 630.

The answer generation system 100 may perform the labeling on each of the extracted molecular structures 611, 621 and 631 so that different labels are assigned to each of the extracted molecular structures 611, 621 and 631. More specifically, the answer generation system 100 may assign the first label M1 to the first content 610 grouped based on the first molecular structure 611 and the second label M2 different from the first label M1 to the second content 620 grouped based on the second molecular structure 621 by labeling the extracted molecular structures 611, 621 and 631. In addition, the answer generation system 100 may assign the third label M3 different from the first label M1 and the second label M2 to the third content 630 grouped based on the third molecular structure 631. In this case, the labeling for molecular structure may be understood as assigning the same label to the content grouped based on the specific molecular structure (i.e., all the content included in the grouped content has the same label).

The grouped content 610, 620 and, 630 to which different labels are assigned as described above may be stored in the storage unit 140 in connection with the user account.

Meanwhile, the answer generation system 100 may provide the grouped content 610, 620, and 630 stored in the storage unit 140 to the user terminal 10 to which the service page 1000 is output.

The answer generation system 100 may provide a graphic object corresponding to each content to which the label is assigned to one region of the service page 1000 where the user query is received. Here, the content to which the label is assigned may correspond to the content (e.g., the image of the molecular structure, the name of the molecular structure, the description of the molecular structure, the SMILES notation of the molecular structure, the properties of the molecular structure, etc.) related to the molecular structure (e.g., the first molecular structure 611, the second molecular structure 621, the third molecular structure 631) extracted from the analysis target document 600 described above.

In this regard, as illustrated in FIG. 7, the service page 1000 may include at least one of a first region 710 in which the information extracted from the analysis target document 600 is provided, a second region 720 in which at least a portion of the document is provided, and a third region 730 in which a user query is received.

First, the first region 710 of the service page 1000 may include at least one grouped content to which the label is assigned. The first region 710 may display information on the extracted (or specified) molecular structure, and such information may be provided in various forms such as the molecular graph, text, or image.

Specifically, the first region 710 may include at least one graphic object 711, 712, and 713 corresponding to the extracted molecular structure (e.g., the first molecular structure 611 to which the first label M1 is assigned, the second molecular structure 621 to which the second label M2 is assigned, and the third molecular structure 631 to which the third label M3 is assigned) to which different labels are respectively assigned through the labeling, and at least one of detailed information on the extracted molecular structures 611, 621 and 631.

The first region 710 of the service page 1000 may include a first sub-region 710a including the graphic objects 711, 712, and 713 and a second sub-region 710b including the detailed information.

In this regard, when the plurality of molecular structures 611, 621 and 631 are extracted from document 600, the first sub-region 710a may include the plurality of graphic objects 711, 712, and 713 corresponding to each of the plurality of molecular structures. More specifically, the first graphic object 711 among the plurality of graphic objects may include the image of the first molecular structure 611 corresponding to the first graphic object 711 among the plurality of molecular structures 611, 621 and 631, and the second graphic object 712 may include the image of the second molecular structure 621 corresponding to the second graphic object 712. In this case, at least some of the graphic objects may include the molecular graph of the molecular structure.

In addition, the detailed information on the molecular structure corresponding to one graphic object selected by the user input among the plurality of graphic objects 711, 712, and 713 may be provided to the second sub-region 710b. The answer generation system 100 may provide the detailed information on the selected graphic object selected to the user input based on receiving the user input for selecting one of the plurality of graphic objects 711, 712, and 713.

In this regard, the detailed information of the molecular structures 611, 621, and 631 corresponding to each of the plurality of graphic objects 711, 712, and 713 may exist in the each of the plurality of graphic objects 711, 712, and 713 included in the first sub-area 710a by being linked (or associated). For example, it is assumed that the first graphic object 711 corresponding to the first molecular structure 611 is selected from the user terminal 10. The answer generation system 100 may provide detailed information 711a, 711b, 711c, 711d, and 711e on the first molecular structure 611 linked to the first graphic object 711 and information on the first label M1 assigned to the first molecular structure to the second sub-region 710b from the user terminal 10, based on the selection of the first graphic object 711 included in the first sub-region 710a.

Here, the detailed information on the molecular structure may include at least one of the molecular structure image 711a of the molecular structure, the name 711b of the molecular structure, the description 711c of the molecular structure 71 1c, the string 711d according to the SMILES notation, and the property 711e of the molecular structure. In this case, the molecular structure image of the molecular structure may also be provided (or displayed) in the form of the molecular graph acquired through the process of acquiring the molecular graph.

Meanwhile, the detailed information (or at least one content included in the grouped content) may be extracted from the document or acquired from at least one pre-trained prediction model. As described above, the pre-trained prediction model may include at least one of the chemical reaction prediction model 400 that predicts the chemical reaction between the molecular structures and the molecular property prediction model 500 that predicts the properties of the molecular structure.

As an example, when the plurality of content extracted from the analysis target document 600 includes the molecular structure image, the name, the description, and the string according to the SMILES notation of the first molecular structure 611, and there are no properties of the first molecular structure 611, the answer generation system 100 may predict the property of the first molecular structure 611 using the pre-trained molecular property prediction model 500. The molecular property prediction model 500 outputs the property prediction result of the first molecular structure 611 as the output data, and the answer generation system 100 may acquire the property prediction result of the first molecular structure 611 and generate the detailed information on the first molecular structure 611. In this case, the molecular structure image 711a, name 711b, description 711c and SMILES notation 711d of the first molecular structure 611 included in the first area 710 of the service page 1000 are extracted from the document 600, and the property 711e of the first molecular structure 611 may be understood to have been generated by the molecular property prediction model 500.

Next, in the second region 720 distinguished from the first region 710 of the service page 1000, the document 600 received from the user terminal 10 may be provided.

Highlighting objects corresponding to each of the molecular structures 611, 621 and 631 may overlap with one region of the document provided on the service page 1000 so that it is identifiable in the second region 720 that the plurality of molecular structures 611, 621 and 631 have been extracted from the document 600. More specifically, highlighting objects 721 and 722 may overlap and displayed in the first region (or first sub-region 720a) including the first molecular structure 611 of the document provided on the service page 1000 and the second region (or second sub-region 720b) including the second molecular structure 621, respectively, so that it is identifiable in the second region 720 that the first molecular structure 611 and the second molecular structure 621 have been extracted from the document 600.

Here, in the first region 720a including the first molecular structure 611, the first label M1 assigned to correspond to the first molecular structure 611 may be provided around the first highlighting object 721 overlapping with the first region 720a. In addition, in the second region 720b including the second molecular structure 621, the second label M2 assigned to correspond to the second molecular structure 621 may be provided around the second highlighting object 722 overlapping with the second region 720b.

Furthermore, the highlighting objects 721 and 722 may be visually highlighted in a user interface (e.g., service page 1000) and thus displayed so as to be distinguished from other objects. For example, the answer generation system 100 may display the highlighting objects 721 and 722 so as to be distinguished from other objects in the service page 1000 by at least one of changing colors, adding borders, and changing background colors of the highlighting object 721 and 722.

Meanwhile, when one highlighting object is selected by the user input, information on a specific molecular structure corresponding to the selected highlighting object may be provided in another region that is distinguished from one region of the service page 1000 where the highlighting object is displayed.

Specifically, the answer generation system 100 may provide detailed information on the specific molecular structure linked to the highlighting object selected according to the user input to the first region 710 of the service page 1000 based on receiving the user input for selecting one of the plurality of highlighting objects 721 and 722. For example, it is assumed that the user input for the first highlighting object 721 of the first region 720a including the first molecular structure 611 is received. The answer generation system 100 may provide detailed information 711a, 711b, 711c, 711d, and 711e on the first molecular structure 611 linked to the first highlighting object 721 to the first region 710 based on receiving the user input for selecting the first highlighting object 721.

In this case, the display of the plurality of graphic objects 711, 712, and 713 included in the first region 710 of the service page 1000 may also be changed according to the selected highlighting object. More specifically, when the first highlighting object 721 is selected, the first graphic object 711 corresponding to the first highlighting object 721 may be highlighted and displayed in the first sub-region 710a of the first region 710 so that the user can identify that the first highlighting object 721 has been selected.

That is, when the highlighting object is selected, the graphic object corresponding to the selected highlighting object may be displayed in the first region 710 with a first visual appearance so that the user may intuitively recognize the graphic object. In contrast, the graphic object corresponding to the unselected highlighting object may be displayed with a second visual appearance.

In this way, as a highlighting object is selected by the user, pieces of information on the specific molecular structure linked to the highlighting object may be provided (or displayed) in another region (first region) that is distinguished from the region (second region) where the highlighting object is displayed.

That is, in the present invention, by visually providing information through graphic objects and labels, the user may easily understand and utilize data related to a complex molecular structure. This may increase the convenience and comprehension of the user and increase the efficiency of research.

Meanwhile, the answer generation system 100 may receive an editing request for the extracted molecular structures through the service page 1000 where the answer to the user query is provided.

The answer generation system 100 may provide a graphic object linked to a function of receiving the editing request for the molecular structures to one region of the service page 1000. For example, as illustrated in FIG. 7, the answer generation system 100 may provide a graphic object 714 linked to the function of receiving the editing request for the molecular structure (first molecular structure 611) corresponding to the molecular structure image to the first region 710 of the service page 1000 where the molecular structure image corresponding to the selected graphic object (first graphic object 711) is displayed.

As another example, although not illustrated, the answer generation system 100 may provide the graphic object linked to the function of receiving the editing request for the molecular structures to each of the plurality of graphic objects 711, 712, and 713 provided to the first region 710. The answer generation system 100 may receive an editing request for a specific molecular structure corresponding to the selected graphic object based on receiving the user input selecting one of the graphic objects provided to each of the plurality of graphic objects 711, 712, and 713.

As another example, although not illustrated, the answer generation system 100 may provide the graphic object linked to the function of receiving the editing request for the molecular structures to each of the plurality of highlighting objects 721 and 722 provided to the second region 720. The answer generation system 100 may receive the editing request for the specific molecular structure corresponding to the selected highlighting object based on receiving the user input selecting one of the graphic objects provided to each of the plurality of highlighting objects 721 and 722.

That is, the present invention does not limit the method of receiving the editing request for the molecular structure. For the convenience of description, the following description will be given on the assumption that the graphic object 714 is selected.

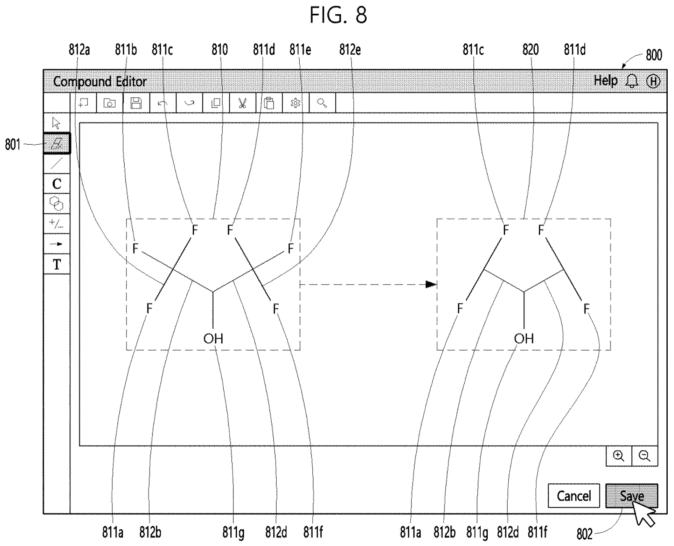

The answer generation system 100 may receive the editing request for the extracted molecular structures (e.g., the first molecular structure) from the user terminal 10 based on the selection of the graphic object 714 included in the first region 710. In addition, as illustrated in FIG. 8, the answer generation system 100 may provide an editing interface 800 providing the editing function for the first molecular structure 611 from the user terminal 10 based on the receipt of the editing request for the first molecular structure 611.

The editing interface 800 may include the molecular structure image of the extracted molecular structure. For example, the answer generation system 100 may provide the molecular structure image 810 of the first molecular structure 611 to the editing interface 800 activated on the user terminal 10 from the user terminal 10 based on receiving the editing request for the first molecular structure 611.

Here, the molecular structure image 810 may also be understood as the molecular graph that includes nodes 811a, 811b, 811c, 811d, 811e, 811f, and 811g corresponding to each of the atoms constituting the extracted first molecular structure 611 and edges 812a, 812b, 812c, 812d, and 812e indicating the bond relationship between the atoms. The first molecular structure 611 may be configured to be edited based on the user input for at least one of nodes 811a, 811b, 811c, 811d, 811e, 811f, and 811g and edges 812a, 812b, 812c, 812d, and 812e.

Specifically, the editing for the extracted first molecular structure 611 may be a deletion or a change in position of at least one of the nodes 811a, 811b, 811c, 811d, 811e, 811f, and 811g corresponding to each of the atoms constituting the extracted molecular structure and the edges 812a, 812b, 812c, 812d, and 812e indicating the bond relationship between the atoms, or may be an addition of a new node corresponding to a new atom or an addition of a new edge generating a new bond relationship between the atoms.

For example, the answer generation system 100 may activate the node and edge deletion mode based on a selection of a graphic object 801 linked to the node and edge deletion function included in one region of the editing interface 800. The answer generation system 100 may edit the first molecular structure 611 such that the specific nodes 811b and 811e at positions corresponding to the user input are deleted based on user input received for selecting the specific nodes 811b and 811e from among the plurality of nodes 811a, 811b, 811c, 811d, 811e, 811f, and 811g included in the image 810 of the first molecular structure.

In this case, when the editing is performed on the first molecular structure 611 as the editing target based on the user input, the molecular structure image corresponding to the edited molecular structure, which is different from the molecular structure image 810 of the first molecular structure 611 before the editing, may be continuously displayed on the editing interface 800. For example, based on the deletion of the specific nodes 811b and 811e corresponding to the user selection, the molecular structure image 820 in which the specific nodes 811b and 811e are deleted may be displayed on the editing interface 800.

Furthermore, the answer generation system 100 may store the extracted molecular structure (or molecular structure image or molecular graph, 820) for which the editing has been performed (or edited) in a predetermined storage (e.g., storage unit 140 or memory) by linking the molecular structure to the user account. For example, the answer generation system 100 may generate the molecular structure 820 in which the first molecular structure 611 is edited based on a selection of a graphic object 802 linked to an editing save (or completion) function included in the editing interface 800 from the user terminal 10, and store the edited molecular structure 820 in the pre-specified storage by linking the edited molecular structure 820 to the user account.

In this way, the present invention may provide a user environment in which a user may design a desired molecule through the editing interface.

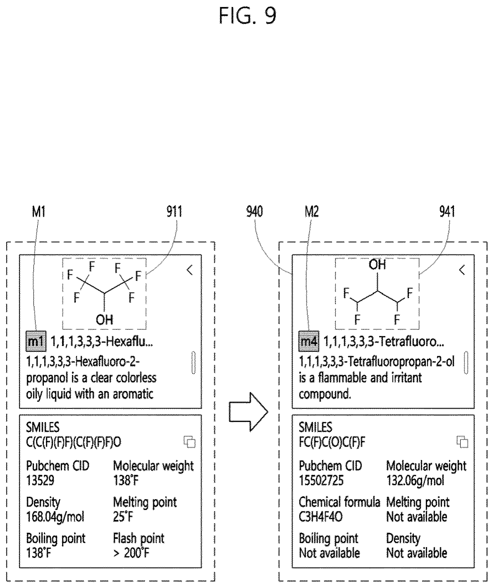

Meanwhile, a new label specifying the edited molecular structure may be assigned to the edited molecular structure. The answer generation system 100 may perform labeling on the edited molecular structure stored in the storage unit 140 so that a new label for specifying the edited molecular structure may be assigned. For example, as illustrated in FIG. 9, the answer generation system 100 may perform labeling on an edited molecular structure 941 so that the edited molecular structure 941 is assigned a fourth label M4, which is different from the first label M1 assigned to the molecular structure (e.g., the first molecular structure 911) before the editing.

The answer generation system 100 may generate a fourth graphic object corresponding to the edited molecular structure 941 and provide the generated fourth graphic object to one region of the service page 1000.

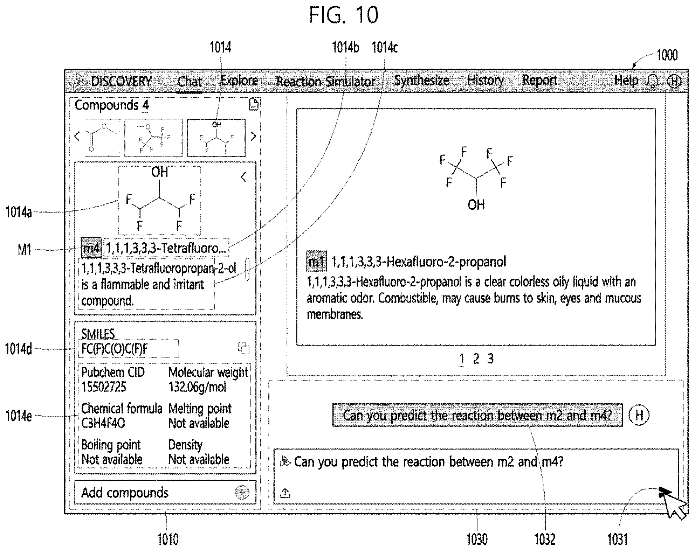

Specifically, as illustrated in FIG. 10, a graphic object (or a fourth graphic object, 1014) corresponding to the edited molecular structure 941 may be provided to the first region (or a first sub-region 1010) of the service page 1000. Here, the graphic object 1014 corresponding to the edited molecular structure 941 may include the molecular structure image of the edited molecular structure 941.

In addition, the first region (or the first sub-region 1010) of the service page 1000 may include a molecular structure image 1014a of the edited molecular structure 941. In addition, the fourth label M4 assigned to the edited molecular structure 941 may also be provided to the surrounding region of the molecular structure image 1014a.

Furthermore, the graphic object 1014 corresponding to the edited molecular structure 941 may further include detailed information on the edited molecular structure 941. For example, the graphic object 1014 may include at least one of a name 1014b of the edited molecular structure 941, a description 1014c of the edited molecular structure 941, a SMILES notation 1014d of the edited molecular structure 941, and a property 1014e of the edited molecular structure 941.

In this case, at least one of the molecular structure image 1014a of the edited molecular structure 941, the name 1014b of the edited molecular structure 941, the description 1014c of the edited molecular structure 941, the SMILES notation 1014d of the edited molecular structure 941, and the property 1014e of the edited molecular structure 941 may be generated by at least one of the pre-trained chemical reaction prediction model 400 and molecular property prediction model 500. For the convenience of description, the edited molecular structure 941 is named "a fourth molecular structure 941", and the detailed information on the edited molecular structure 941 is named "the grouped fourth content 940" (see FIG. 9).

Meanwhile, in the present invention, a process (S430) of receiving the user query from the user terminal may be performed (see FIG. 4).

The answer generation system 100 may receive a user query including at least one of the labels assigned by the labeling through the service page 1000.

As illustrated in FIG. 10, a third region 1030 of the service page 1000 may be configured to receive the user query. The third region 1030 may include a graphic object 1030a linked to the function of receiving the user query.

The answer generation system 100 may receive a user query including a label assigned to a specific molecular structure through the third region 1030 of the service page 1000. For example, the answer generation system 100 may receive a user query 1032 (e.g., "Can you predict the reaction between m2 and m4?") including the second label M2 assigned to the second molecular structure 621 and the fourth label M4 assigned to the edited molecular structure 941 from the user terminal 10, based on the selection of the graphic object 1031 included in the third region 1030.

In other words, the user may input a query more intuitively and simply by utilizing the label assigned to the specific molecular structure without having to input complex information on the specific molecular structure.

When the user query is received, the present invention may perform a process (S440) of specifying specific content related to the user query among the plurality of content (see FIG. 4).

The answer generation system 100 may input the user query 1032 to the ultra-large foundation model 200 based on receiving the user query 1032.

The ultra-large foundation model 200 may receive the user query 1032 as the input, understand the query (or contents) included in the user query 1032, and specify the specific content related to the user query 1032. More specifically, the ultra-large foundation model 200 may analyze the user query 1032 and extract the label indicating the grouped content from user query 1032. For example, the ultra-large foundation model 200 may extract the second label M2 and the fourth label M4 as the result of analyzing the user query 1032, based on the fact that the text corresponding to the second label M2 indicating the grouped second content 620 and the fourth label M4 indicating the grouped fourth content 940 are included in the user query 1032.

Furthermore, the ultra-large foundation model 200 may specify the specific grouped content corresponding to the extracted label. For example, the ultra-large foundation model 200 may specify the grouped second content 620 corresponding to the extracted second label M2 and the grouped fourth content 940 corresponding to the extracted fourth label M4 as the specific grouped content.

In this way, in the present invention, the time required to identify the specific content corresponding to the user query may be shortened through the label assigned to the extracted molecular structure.

When the content related to the user query is specified, in the present invention, a process (S450) of processing the specified content as the input to the pre-trained prediction model may be performed (see FIG. 4).

The ultra-large foundation model 200 may process the molecular structure of the specific grouped content as the input to the pre-trained prediction model. More specifically, the ultra-large foundation model 200 may process the grouped second content 620 to which the second label M2 is assigned and the grouped fourth content 940 to which the fourth label M4 is assigned as the inputs to the pre-trained prediction model.

In this case, it may be determined based on the user query 1032 which of the multiple pre-trained prediction models to process the specified content as input. For example, the ultra-large foundation model 200 may understand the content included in the user query 1032, and may determine that the user query 1032 is related to the chemical reaction prediction for the plurality of molecular structures based on the fact that the user query 1032 includes the content "Can you predict the chemical reaction between m2 and m4?" Based on the determination results, the ultra-large foundation model 200 may determine the prediction model to which the specific grouped second content 620 and fourth content 940 will be input as the chemical reaction prediction model 400.

Furthermore, the ultra-large foundation model 200 may process the specific grouped second content 620 and fourth content 940 as inputs to the chemical reaction prediction model 400. The ultra-large foundation model 200 may convert the names of the second molecular structure 621 and the fourth molecular structure 941 included in each of the specific grouped second content 620 and fourth content 940 into strings according to the SMILES notation, which is a language that the computer may understand, and input the converted strings and the information on the specific grouped second content 620 and fourth content 940 to the chemical reaction prediction model 400 that understands the chemical reaction mechanisms.

When the output data is acquired from the pre-trained prediction model, in the present invention, a process (S460) of generating the answer to the user query using the output data of the prediction model may be performed (see FIG. 4).

As described above, the chemical reaction prediction model 400 may receive the string converted from the ultra-large foundation model 200 and the information on the specific grouped second content 620 and fourth content 940. The chemical reaction prediction model 400, which has received the information, may predict the chemical reaction between the second molecular structure 621 corresponding to the grouped second content 620 and the fourth molecular structure 941 corresponding to the grouped fourth content 940, and output the predicted results of the chemical reaction between the second molecular structure 621 and the fourth molecular structure 941 as the output data.

Here, the output data of the chemical reaction prediction model 400 may include the specific molecular structure generated as the predicted results of the chemical reaction between the plurality of molecular structures and at least one piece of information (or content) related to the specific molecular structure. For example, the output data may include at least one of a fifth molecular structure generated as the predicted results of the chemical reaction between the second molecular structure 621 and the fourth molecular structure 941, the molecular structure image of the fifth molecular structure, a name of the fifth molecular structure, a description of the fifth molecular structure, and a SMILES notation of the fifth molecular structure.

Meanwhile, the ultra-large foundation model 200 may determine the answer generation procedure performed for prediction corresponding to the user query 1032 and the tool used for the answer generation procedure. For example, as illustrated in FIG. 11, the ultra-large foundation model 200 may determine what procedure and tool to use to generate the answer to the user query 1032.

The answer generation system 100 may provide the information on the answer generation procedure and tool determined from the ultra-large foundation model 200 to the service page 1000. For example, the answer generation system 100 may provide information 1101 on the answer generation procedure and tool determined from the ultra-large foundation model 200 through the service page 1000 to perform a prediction corresponding to a user query 1100 (e.g., "Can you predict the reaction between m2 and m4?").

Furthermore, the ultra-large foundation model 200 may generate the answer to the user query 1032 using the molecular structures (e.g., the second molecular structure 621 and the fourth molecular structure 941) corresponding to the specific labels (the second label M2 and the fourth label M4) included in the user query 1032 among the extracted molecular structures. More specifically, the ultra-large foundation model 200 may generate an answer 1110 to the user query 1100 using the output data of the chemical reaction prediction model 400 and the contents (e.g., contents related to the second molecular structure 621 and the fourth molecular structure 941) constituting the grouped content and the information 1101 on the determined answer generation procedure and tool.

Meanwhile, the answer generation system 100 may predict the properties of the specific molecular structure using the pre-trained molecular property prediction model 500, and also provide the information on the properties of the specific molecular structure predicted from the molecular property prediction model 500 as the answer 1110 to the user query 1100.

As described above, the molecular property prediction model 500 may be a model built for material structure design. The molecular property prediction model 500 may be configured to predict the physical properties from the molecular structure or design a molecule having the user's desired characteristics (or new characteristics).

Specifically, the answer generation system 100 may process the fifth molecular structure as the input to the molecular property prediction model 500. The molecular property prediction model 500 may receive the fifth molecular structure as an input and output the property prediction result of the fifth molecular structure as output data. The answer generation system 100 may acquire the property prediction result of the fifth molecular structure output from the molecular property prediction model 500.

Furthermore, the answer generation system 100 may input the property prediction result of the fifth molecular structure to the ultra-large foundation model 200. The ultra-large foundation model 200 may generate the answer 1110 to the user query 1100 using the property prediction result of the fifth molecular structure. In this case, the answer generation system 100 may provide the information on the physical properties of the fifth molecular structure predicted from the molecular property prediction model 500 as the answer 1110 to the user query 1100.

Meanwhile, when the answer 1110 to the user query 1100 includes the specific molecular structure (or a new molecular structure) generated through the pre-trained prediction model, a label may be assigned to the specific molecular structure.

The answer generation system 100 may perform labeling on the specific molecular structure so that a new label is assigned to specify the specific molecular structure. For example, the answer generation system 100 may perform the labeling on the fifth molecular structure so that the fifth label M5 is assigned to the fifth molecular structure generated through the chemical reaction prediction model 400.

Here, the specific molecular structure (the fifth molecular structure) and the label assigned to the specific molecular structure (the fifth label M5) may be stored in the pre-specified storage by being linked to the user account, together with the extracted molecular structure and the label assigned to the extracted molecular structure.

In addition, the answer generation system 100 may generate a specific graphic object corresponding to the specific molecular structure based on the fact that the specific molecular structure is generated from the pre-trained prediction model. For example, the answer generation system 100 may generate the fifth graphic object corresponding to the fifth molecular structure using the fifth molecular structure stored in the storage unit 140.

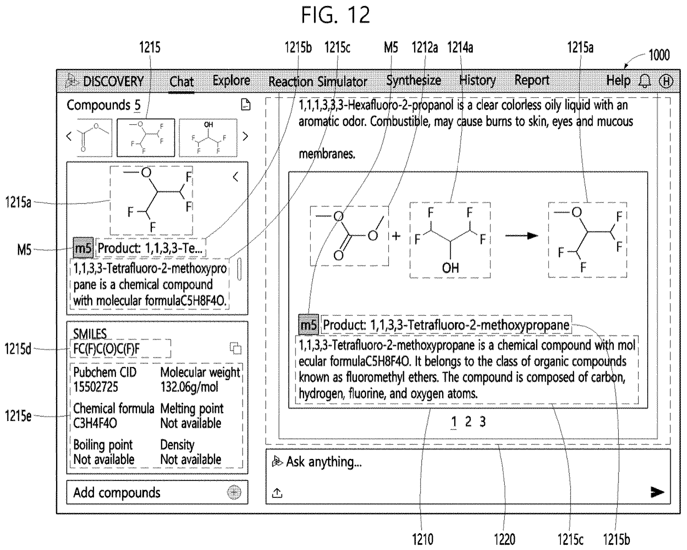

Furthermore, the answer generation system 100 may perform an update on the service page 1000 so that the specific graphic object corresponding to the specific molecular structure is included in the region of the service page 1000. More specifically, as illustrated in FIG. 12, the answer generation system 100 may perform an update on a first region 1210 so that a graphic object (or a fifth graphic object 1215) corresponding to the fifth molecular structure is included in the first region 1210 of the service page 1000.

Based on the update, the detailed information on the molecular structure corresponding to the specific graphic object may be provided to the first region 1210 of the service page 1000 together with the specific graphic object. For example, the first region 1210 may include a molecular structure image 1215a of the fifth molecular structure corresponding to the fifth graphic object 1215, a name 1215b of the fifth molecular structure, a description 1215c of the fifth molecular structure, a SMILES notation 1215d of the fifth molecular structure, and a property 1215e of the fifth molecular structure.

Meanwhile, the answer generation system 100 may provide an answer 1221 (e.g., "The product generated through the chemical reaction between m2 and m4 is m5...,") to the user query 1100 generated from the ultra-large foundation model 200 to one region (or the second region, 1220) of the service page 1000.

The answer 1221 provided to the second region 1220 of the service page 1000 may include contents indicating that a new specific molecular structure (e.g., the fifth molecular structure) is generated as the predicted results of the chemical reaction between the plurality of molecular structures (e.g., the second molecular structure 621 and the fourth molecular structure 941).

Specifically, the answer 1221 may include a molecular structure image 1212a of the second molecular structure 621 and a molecular structure image 1214a of the fourth molecular structure 941, and may include a molecular structure image 1215a of the fifth molecular structure generated as the results of the chemical reaction between the second molecular structure 621 and the fourth molecular structure 941.

In addition, graphic objects (e.g., a plus sign, an arrow, etc.) indicating the relationship between the molecular structures corresponding to each image may also be displayed between the image 1212a of the second molecular structure, the image 1214a of the fourth molecular structure, and the image 1215a of the fifth molecular structure that are included in the answer 1221.

Furthermore, the answer 1221 may include at least one of the detailed information (e.g., a name 1215b of the fifth molecular structure, a description 1215c of the fifth molecular structure, etc.) on the fifth molecular structure generated through the chemical reaction prediction model 400.

Furthermore, the information on the properties of the fifth molecular structure predicted from the property prediction model 500 may be also provided to the answer 1221, and the information on the fifth label M5 assigned to the fifth molecular structure may also be displayed to the surrounding region of the molecular structure image 1215a of the fifth molecular structure.

Meanwhile, the answer generation system 100 may receive a new (or added) user query including the label assigned to the specific molecular structure (e.g., fifth molecular structure) through the service page 1000.

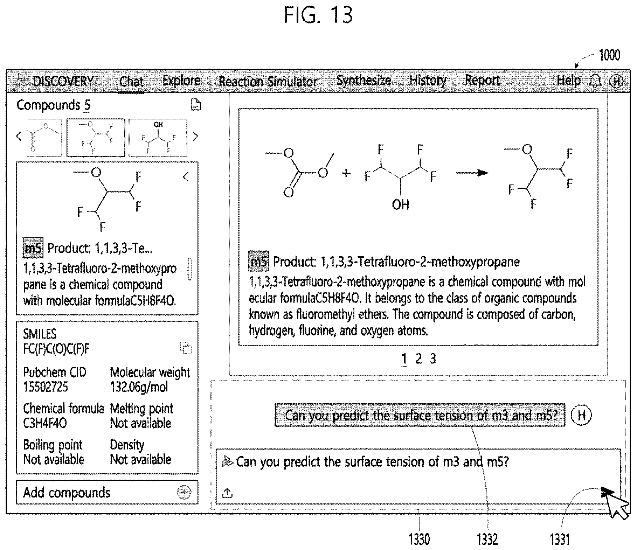

Specifically, the answer generation system 100 may receive a new user query including a label assigned to a specific molecular structure through the third region 1030 of the service page 1000. For example, as illustrated in FIG. 13, the answer generation system 100 may receive a new user query 1332 (e.g., "Can you predict the surface tension of m3 and m5?") including the third label M3 assigned to the third molecular structure 631 and the fifth label M5 assigned to the fifth molecular structure from the user terminal 10 based on a selection of a graphic object 1331 included in a third region 1330.

The answer generation system 100 may input the new user query 1332 to the ultra-large foundation model 200 based on receiving the new user query 1332 including the label assigned to the fifth molecular structure.

The ultra-large foundation model 200 may generate the answer to the new user query 1332 using at least some of the information on the specific molecular structure and the property of the specific molecular structure corresponding to the label assigned to the specific molecular structure. For example, the ultra-large foundation model 200 may generate the answer to the new user query 1332 using at least some of the information on the third molecular structure 631 corresponding to the third label M3, the fifth molecular structure corresponding to the fifth label M5, and the properties of the third molecular structure 631 and the fifth molecular structure.

In this case, the ultra-large foundation model 200 may utilize at least one prediction model to generate the answer to the new user query 1332. For example, the ultra-large foundation model 200 may understand the contents included in the new user query 1332 and input the information on the properties of the third molecular structure 631, the fifth molecular structure, the third molecular structure 631, and the fifth molecular structure to the molecular property prediction model 500 based on the fact that the user query 1332 includes the contents "Can you predict the surface tension of m3 and m5?"

The molecular property prediction model 500 may predict the properties (e.g., surface tension) for the third molecular structure 631 and the fifth molecular structure corresponding to the new user query 1332. The molecular property prediction model 500 may output the property prediction results of the third molecular structure 631 and the fifth molecular structure as the output data.

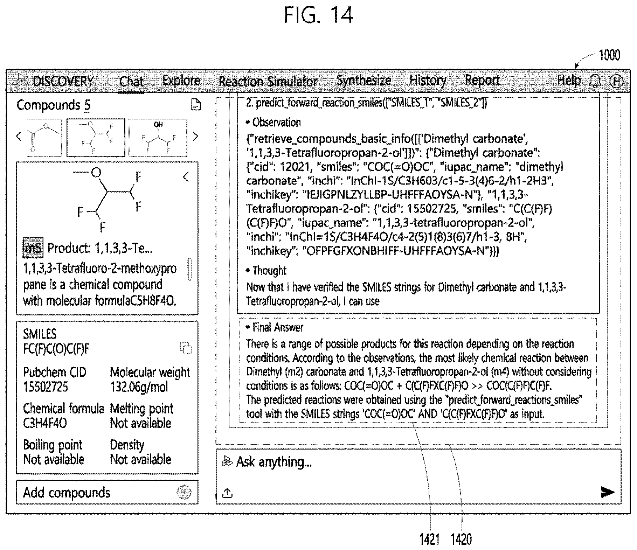

The ultra-large foundation model 200 may determine the answer generation procedure performed for prediction corresponding to the new user query 1332 and the tool used in the answer generation procedure, and generate an answer 1421 (e.g., "surface tension of m3 is OO, surface tension of m5 is OO...,") to the user query 1332 using the determined answer generation procedure and tool, the output data of the molecular property prediction model 500, and the information on the third molecular structure and the fifth molecular structure (see FIG. 14).

When the answer 1421 is generated, as illustrated in FIG. 14, the answer generation system 100 may provide the answer 1421 to the user query 1100 generated from the ultra-large foundation model 200 to one region (or a second region, 1420) of the service page 1000. In this case, the answer generation system 100 may perform the update on the first region so that the properties (e.g., surface tension) of the third molecular structure 631 and the fifth molecular structure predicted from the molecular property prediction model 500 are displayed in the first region.

Meanwhile, the answer generation method of the ultra-large foundation model described above was described on the premise that the document was received, but the answer generation method of the ultra-large foundation model will be described below in more detail on the premise that the text was received.

First, the answer generation system 100 may receive the user query in the form of the text from the user terminal 10 to which the service page 1000 is provided.

Specifically, the answer generation system 100 may receive the user query including the information on at least one molecular structure through the region of the service page 1000. Here, the information on the molecular structure included in the user query may exist variously. For example, the information on the molecular structure may include the name of the specific molecular structure, the description of the specific molecular structure, the SMILES notation of the specific molecular structure, the formula of the specific molecular structure, etc. However, the information on the molecular structure described above is only an example, and the information on the molecular structure included in the user query in the present invention is not necessarily limited thereto, and it is obvious that it may further include various types of information related to the molecular structure.

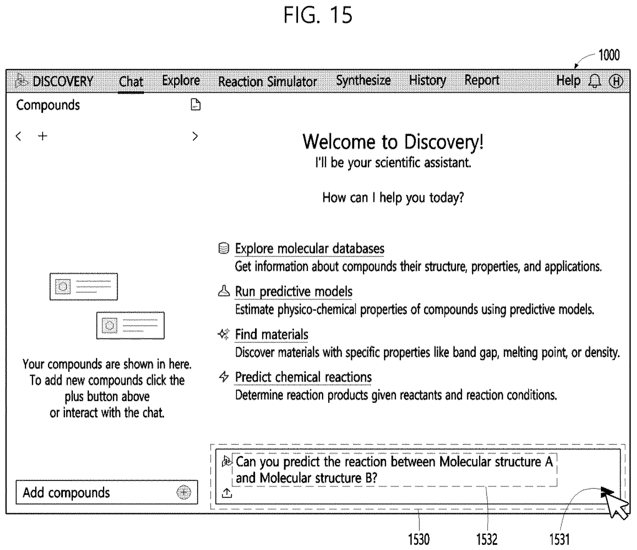

For example, as illustrated in FIG. 15, the answer generation system 100 may receive a user query 1532 (e.g., "Can you predict the reaction between Molecular structure A and Molecular structure B?") including a name (e.g., "Molecular structure A," "Molecular structure B") of a specific molecular structure based on a selection of a graphic object 1531 included in a third region 1530 from the user terminal 10.

The answer generation system 100 may input the user query 1532 to the ultra-large foundation model 200 based on receiving the user query 1532.

The ultra-large foundation model 200 may specify the specific molecular structure corresponding to the name of the specific molecular structure based on the name of the specific molecular structure included in the user query 1532. For example, the ultra-large foundation model 200 may specify the first molecular structure and the second molecular structure corresponding to each of the specific molecular structures from the name (e.g., "Molecular structure A", "Molecular structure B") of the specific molecular structure included in the user query 1532.

When the molecular structure is specified, the answer generation system 100 may extract (or generate) a plurality of content including the contents related to the specified molecular structure. In this case, the information on the specified molecular structure may also be extracted by the ultra-large foundation model 200.

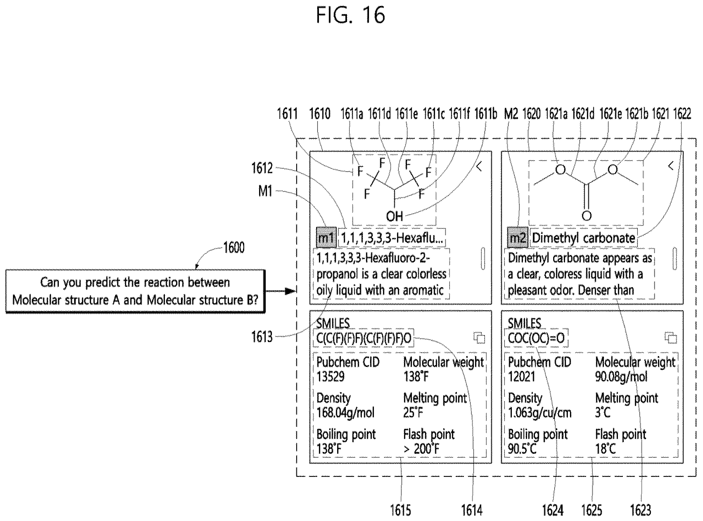

For example, as illustrated in FIG. 16, the answer generation system 100 may extract at least one of a molecular structure image of a first molecular structure 1611, a name 1612 of the first molecular structure, a description 1613 of the first molecular structure, a SMILES notation 1614 of the first molecular structure, and a property 1614 of the first molecular structure that are related to the first molecular structure specified from a user query 1600. In addition, the answer generation system 100 may extract at least one of a molecular structure image 1621 of a second molecular structure, a name 1622 of the second molecular structure, a description 1623 of the second molecular structure, a SMILES notation 1624 of the second molecular structure, and a property 1625 of the first molecular structure that are related to the second molecular structure.

Here, the plurality of content may be i) extracted from contents (or information or data) related to various molecular structures stored in the storage unit 140, or ii) generated by at least one of the chemical reaction prediction model 400 and the molecular property prediction model 500.

As an example, the information related to various molecular structures stored in the storage unit 140 may include contents related to molecular structures related to at least one of the chemistry, the bio, the new materials, the new substances, and the new drug development extracted from each of the plurality of documents using the document understanding model 300. The ultra-large foundation model 200 may extract, as content, at least one contents related to the specified molecular structure from among the contents related to the molecular structure stored in the storage unit 140.

As another example, when the specific molecular structure is specified from the user query, the ultra-large foundation model 200 may generate at least one content related to the specified molecular structure using at least one of the pre-trained chemical reaction prediction model 400 and molecular property prediction model 500.

The answer generation system 100 may group contents about the same molecular structure among the plurality of content into content related to each other based on the relationship between the plurality of content. More specifically, the answer generation system 100 may group, into the content related to the first molecular structure, a molecular structure 1611 image of the first molecular structure, a name 1612 of the first molecular structure, a description 1613 of the first molecular structure, a SMILES notation 1614 of the first molecular structure, and a property 1615 of the first molecular structure, which include contents related to the first molecular structure. In addition, the answer generation system 100 may group, into the content related to the second molecular structure 621, a molecular structure image 1621 of the second molecular structure, a name 1622 of the second molecular structure, a description 1623 of the second molecular structure, a SMILES notation 1624 of the second molecular structure, and a property 1625 of the first molecular structure, which include contents related to the second molecular structure.

Through the grouping process described above, the content grouped based on the molecular structure may be generated. For example, the first molecular structure, the molecular structure image 1611 of the first molecular structure, the name 1612 of the first molecular structure, the description 1613 of the first molecular structure, the SMILES notation 1614 of the first molecular structure, and the property 1615 of the first molecular structure may be grouped to generate the grouped first content 1610. In addition, the second molecular structure, the molecular structure image 1621 of the second molecular structure, the name 1622 of the second molecular structure, the description 1623 of the second molecular structure, the SMILES notation 1624 of the second molecular structure, and the property 1625 of the first molecular structure may be grouped to generate the grouped second content 1620.

Furthermore, the answer generation system 100 may perform labeling on each of the grouped contents 1610 and 1620, such that a label is assigned to each of the grouped contents 1610 and 1620.

More specifically, the answer generation system 100 may perform different labeling on each of the grouped first content 1610 and the second content 1620, so that different labels may be assigned to each of the grouped first content 1610 and second content 1620. For example, the answer generation system 100 may assign the first label M1 to the grouped first content 1610 based on the first molecular structure, and may assign the second label M2 different from the first label M1 to the grouped second content 1620 based on the second molecular structure.

Meanwhile, the ultra-large foundation model 200 may understand the contents included in the user query 1600, and may determine the prediction model to which the grouped first content 1610 and second content 1620 will be input as the chemical reaction prediction model 400 based on the fact that the user query 1032 includes the contents "Can you predict the reaction between molecular structure A and molecular structure B?"

The ultra-large foundation model 200 may process the grouped first content 1610 and second content 1620 as the inputs to the chemical reaction prediction model 400. The chemical reaction prediction model 400 may predict the chemical reaction between the first molecular structure corresponding to the grouped first content 1610 and the second molecular structure corresponding to the grouped second content 1620, and output the predicted results of the chemical reaction between the first molecular structure and the second molecular structure as the output data.

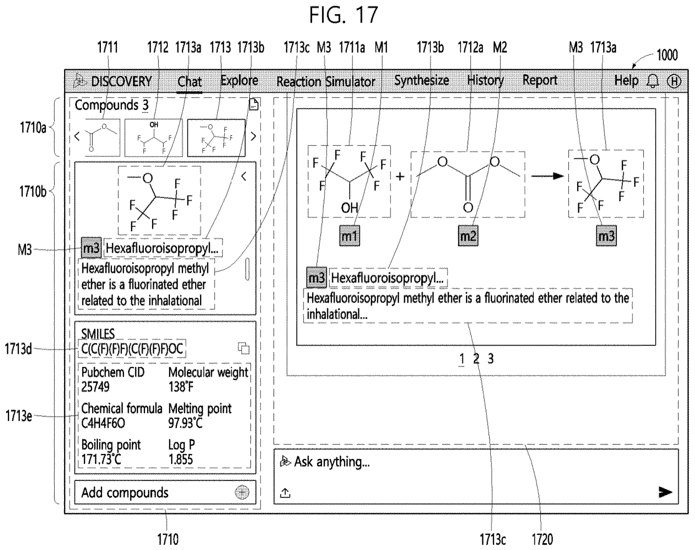

Furthermore, the ultra-large foundation model 200 may generate an answer 1721 to the user query 1600 (e.g., the product generated through the chemical reaction between m1 and m2 is m3...",) by using the output data of the chemical reaction prediction model 400 and the contents (e.g., the contents about the first molecular structure and the second molecular structure) constituting the grouped content, and the information on the determined answer generation procedure and tool (see FIG. 17).

Meanwhile, the answer generation system 100 may generate the third content (or detailed information) grouped based on the specific molecular structure based on the fact that the answer 1721 to the user query 1600 includes the specific molecular structure (or a new molecular structure). For the convenience of description, the specific molecular structure will be described below by naming "the third molecular structure."

In this case, at least some of the information included in the grouped third content may be generated by at least one of the chemical reaction prediction model 400 and the molecular property prediction model 500. As another example, at least some of the information included in the grouped third content may be generated using the information related to various molecular structures stored in the storage unit 140.

In addition, the answer generation system 100 may perform the labeling on the grouped third content based on the third molecular structure so that a new label for specifying the third molecular structure is assigned. For example, the answer generation system 100 may perform the labeling on the third molecular structure so that the third label M3 is assigned to the third molecular structure generated through the chemical reaction prediction model 400.

Here, the third molecular structure and the third label assigned to the third molecular structure may be stored in the pre-specified storage by being linked to the user account, together with the extracted molecular structure (e.g., the first molecular structure and the second molecular structure) and the label (e.g., the first label and the second label) assigned to the extracted molecular structure.

In addition, the answer generation system 100 may generate the specific graphic object corresponding to the third molecular structure. For example, the answer generation system 100 may generate a third graphic object corresponding to the third molecular structure generated through the chemical reaction prediction model 400.

Meanwhile, as illustrated in FIG. 17, the answer generation system 100 may provide the graphic objects corresponding to each content to which the labels are assigned, together with the answer 1721 generated from then ultra-large foundation model 200, to the service page 1000. Here, the content to which the label is assigned may include the grouped first content 1610 and second content 1620, and the third content grouped based on the third molecular structure included in the answer 1720 to the user query 1600.

First, the first region 710 of the service page 1000 may include at least one grouped content to which the label is assigned.

Specifically, the first region 1710 may include at least one graphic object 1712, 1713, and 713 corresponding to the plurality of molecular structures (e.g., the first molecular structure 611 to which the first label M1 is assigned, the second molecular structure 621 to which the second label M2 is assigned, and the third molecular structure 631 to which the third label M3 is assigned) to which different labels are respectively assigned through the labeling, and at least one of detailed information on the plurality of molecular structures 611, 621 and 1711.

The first region 710 of the service page 1000 may include a first sub-region 1,710a including the graphic objects 1711, 1712, and 1713 and a second sub-region 1710b including the detailed information.

The first sub-region 1710a may include the plurality of graphic objects 1711, 1712, and 1713 corresponding to each of the plurality of molecular structures. More specifically, the first graphic object 1711 among the plurality of graphic objects may include the image of the first molecular structure corresponding to the first graphic object 1711 among the plurality of molecular structures, and the third graphic object 1713 may include the image of the third molecular structure corresponding to the third graphic object 1713.

In addition, the detailed information on the molecular structure corresponding to one graphic object selected by the user input among the plurality of graphic objects 1711, 1712, and 1713 may be provided to the second sub-region 1710b. The answer generation system 100 may provide the detailed information on the selected graphic object selected to the user input based on receiving the user input for selecting one of the plurality of graphic objects 1711, 1712, and 1713.

In this regard, the detailed information of the molecular structures corresponding to each of the plurality of graphic objects 1711, 1712, and 1713 may exist in the each of the plurality of graphic objects 1711, 1712, and 1713 included in the first sub-area 1710a by being linked (or associated). For example, it is assumed that the third graphic object 1713 corresponding to the third molecular structure 611 is selected from the user terminal 10. The answer generation system 100 may provide detailed information 1711a, 1711b, 1711c, 1711d, and 1711e on the third molecular structure linked to the third graphic object 1713 to the second sub-region 1710b from the user terminal 10, based on the selection of the third graphic object 1713 included in the first sub-region 710a. Furthermore, the information on the third label M3 assigned to the third molecular structure may also be displayed in the second sub-region 1710b.

As another example, when a new molecular structure (e.g., the third molecular structure) is included in the answer to the user query, the answer generation system 100 may automatically select the graphic object corresponding to the new molecular structure included in the first sub-region 1710a to provide the detailed information on the new molecular structure to the second sub-region 1710b.

Next, an answer 1721 to the user query 1600 may be provided to the second region 1720 that is distinguished from the first region 1710 of the service page 1000.

The answer 1721 provided to the second region 1720 of the service page 1000 may include contents indicating that a new specific molecular structure (e.g., the third molecular structure) is generated as the predicted results of the chemical reaction between the plurality of molecular structures (e.g., the first molecular structure and the second molecular structure).

Specifically, the answer 1721 may include the molecular structure image 1711a of the first molecular structure and a molecular structure image 1712a of the second molecular structure, and may include a molecular structure image 1713a of the third molecular structure generated as the results of the chemical reaction between the first molecular structure and the second molecular structure.

In addition, the graphic objects (e.g., a plus sign, an arrow, etc.) indicating the relationship between the molecular structures corresponding to each image may also be displayed between the molecular structure image 1711a of the first molecular structure, the molecular structure image 1712a of the second molecular structure and the molecular structure image 1713a of the third molecular structure that are included in the answer 1721.

Furthermore, the answer 1721 may include at least one of the detailed information (e.g., a name 1713b of the third molecular structure, a description 1713c of the third molecular structure, etc.) on the third molecular structure generated through the chemical reaction prediction model 400.

Furthermore, the information on the property of the third molecular structure predicted from the property prediction model 500 may be also provided to the answer 1221, and the information on the third label M3 assigned to the third molecular structure may also be displayed to the surrounding region of the molecular structure image 1713a of the third molecular structure.

Meanwhile, the answer generation system 100 may receive a new user query including the third label M3 assigned to the third molecular structure through the service page 1000.

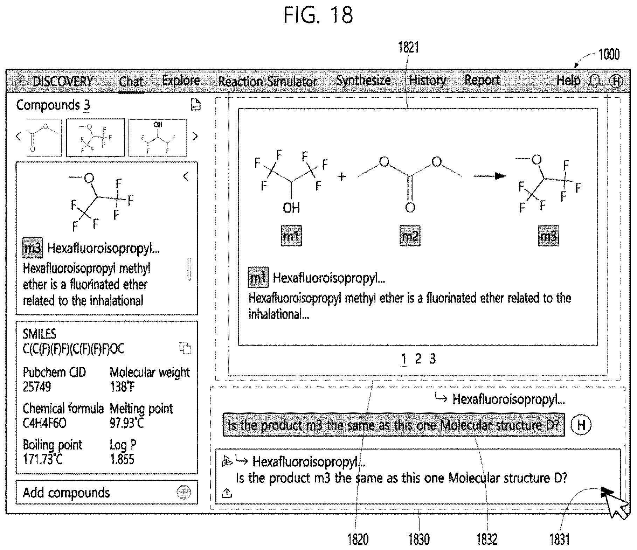

Specifically, the answer generation system 100 may receive a new user query including the third label M3 assigned to the third molecular structure through the third region of the service page 1000. For example, as illustrated in FIG. 18, the answer generation system 100 may receive a new user query 1832 (e.g., "Is the product m3 the same as this one Molecular structure D?") including the third label M3 assigned to the third molecular structure from the user terminal 10 based on a selection of a graphic object 1831 included in a third region 1830.

The answer generation system 100 may input the new user query 1832 to the ultra-large foundation model 200 based on receiving the new user query 1832 including the label M3 assigned to the third molecular structure.

Here, the ultra-large foundation model 200 may specify the fourth molecular structure corresponding to the name of the specific molecular structure and extract the contents related to the fourth molecular structure based on the new user query 1832 including the name (e.g., "Molecular structure D") of the new specific molecular structure instead of the content to which the label is assigned. Then, the answer generation system 100 may group the contents related to the fourth molecular structure, generate fourth content grouped based on the fourth molecular structure, and assign the fourth label M4 to the grouped fourth content. More specific details related to this have been described above, and therefore, will be described briefly.

The ultra-large foundation model 200 may generate the answer to the new user query 1832 using at least some of the detailed information of the third molecular structure corresponding to the third label M3 and the fourth molecular structure corresponding to the fourth label M4. For example, the ultra-large foundation model 200 may generate an answer to the new user query 1832 using at least some of the information included in the grouped third content to which the third label M3 is assigned and the grouped fourth content to which the fourth label M4 is assigned.

In this case, the ultra-large foundation model 200 may utilize at least one prediction model to generate the answer to the new user query 1832. For example, the ultra-large foundation model 200 may understand the contents included in the new user query 1832 and input the information on the third content grouped based on the third molecular structure and the fourth content grouped based on the fourth molecular structure to the chemical reaction prediction model 400 based on the fact that the user query 1832 includes the contents "Do m3 and molecular structure D have the same structure?"

The chemical reaction prediction model 400 may compare the connection structures of the third molecular structure and the fourth molecular structure corresponding to the new user query 1832. The chemical reaction prediction model 400 may output the comparison results for the third molecular structure and the fourth molecular structure as the output data.

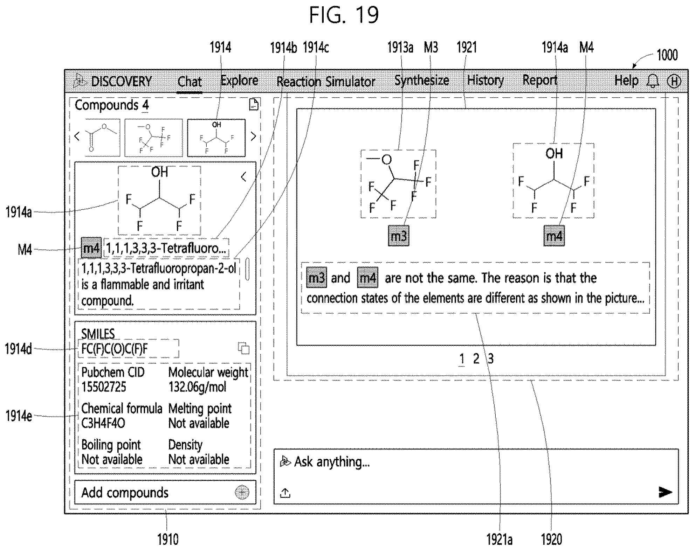

Meanwhile, the ultra-large foundation model 200 may determine the answer generation procedure performed for prediction corresponding to the new user query 1832 and the tool used in the answer generation procedure, and generate an answer 1921 (e.g., "m3 and m4 are connected in different relationships...") to the user query 1832 using the determined answer generation procedure and tool, the output data of the chemical reaction prediction model 400, and the information on the third molecular structure and the fourth molecular structure (see FIG. 19).

When the answer 1921 is generated, as illustrated in FIG. 19, the answer generation system 100 may provide the answer 1921 to the user query 1832 generated from the ultra-large foundation model 200 to a second region 1920 of the service page 1000.

In this case, the answer 1921 provided to the second region 1920 of the service page 1000 may include contents indicating the results of comparison of the connection structures between the plurality of molecular structures (e.g., the third molecular structure and the fourth molecular structure). For example, the answer 1921 may include at least one of labels M3 and M4 assigned to each of the third molecular structure and the fourth molecular structure, molecular structure images 1913a and 1914a of each of the third molecular structure and the fourth molecular structure, and contents 1921a describing the results of comparison of connection structures between the third molecular structure and the fourth molecular structure.

Furthermore, the first region 1910 of the service page 1000 may include at least one of a fourth graphic object 1914 corresponding to the fourth molecular structure, the molecular structure image 1914a of the fourth molecular structure linked to the fourth graphic object 1914, a name 1914b of the fourth molecular structure, a description 1914c of the fourth molecular structure, a SMILES notation 1914d for the fourth molecular structure, and a property 1914e of the fourth molecular structure.

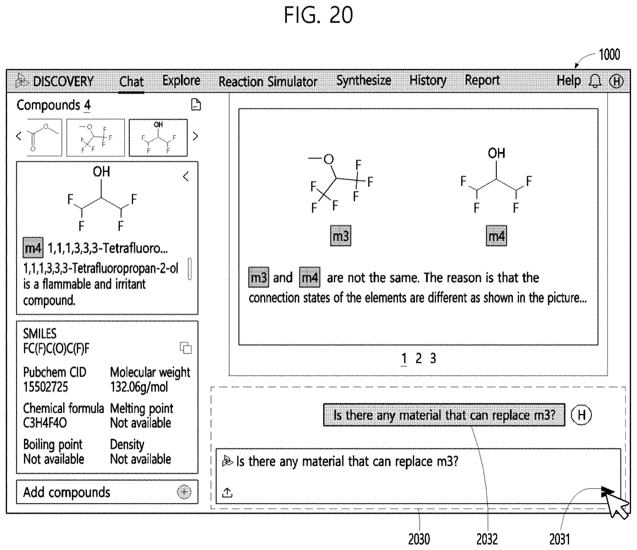

Next, the answer generation system 100 may receive a new user query including the third label M3 assigned to the third molecular structure through the third region of the service page 1000. For example, as illustrated in FIG. 20, the answer generation system 100 may receive a new user query 2032 (e.g., "Is there any material that can replace m3?") including the third label M3 assigned to the third molecular structure from the user terminal 10 based on a selection of a graphic object 2031 included in a third region 2030.

The answer generation system 100 may input the new user query 2032 to the ultra-large foundation model 200 based on receiving the new user query 2032 including the label M3 assigned to the third molecular structure.

The ultra-large foundation model 200 may generate the answer to the new user query 2032 using the third molecular structure corresponding to the third label M3 and at least some of the detailed information of the third molecular structure. For example, the ultra-large foundation model 200 may generate an answer to the new user query 2032 using at least some of the information included in the grouped third content to which the third label M3 is assigned.

In this case, the ultra-large foundation model 200 may utilize at least one prediction model to generate the answer to the new user query 2032. For example, the ultra-large foundation model 200 may understand the contents included the new user query 2032 and input the information on the third content grouped based on the third molecular structure, based on the fact that the user query 2032 includes the contents "So what are the molecular structures can replace m3?" to the chemical reaction prediction model 400.

The chemical reaction prediction model 400 may predict a specific molecular structure that may replace the third molecular structure corresponding to the new user query 2032. In addition, the chemical reaction prediction model 400 may output the predicted results of the specific molecular structure that can replace the third molecular structure as the output data.

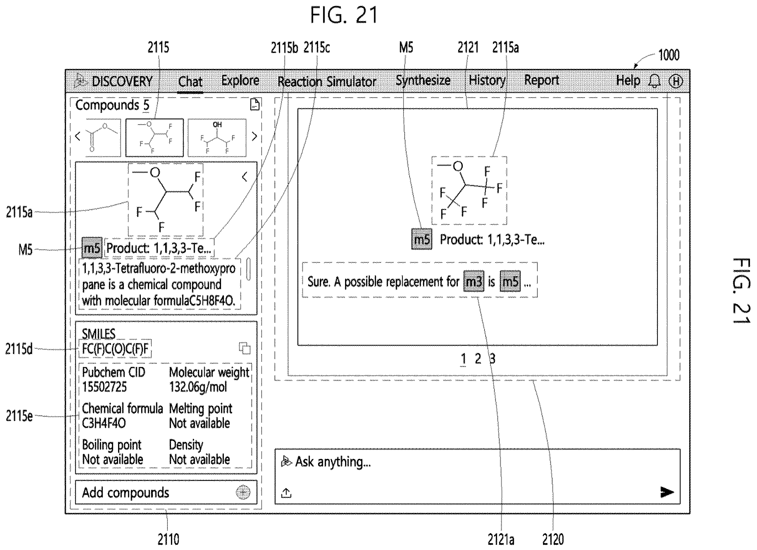

Meanwhile, the ultra-large foundation model 200 may determine the answer generation procedure performed for prediction corresponding to the new user query 2032 and the tool used in the answer generation procedure, and generate an answer 2121 (e.g., "The molecular structure that can replace m3 is m5...") to the user query 2032 using the determined answer generation procedure and tool, the output data of the chemical reaction prediction model 400, and the information on the third molecular structure (see FIG. 21).

When the answer 2121 is generated, as illustrated in FIG. 21, the answer generation system 100 may provide the answer 2121 to the user query 2032 generated from the ultra-large foundation model 200 to a second region 2120 of the service page 1000.

In this case, the answer 1921 provided in the second region 1920 of the service page 1000 may include the contents on the specific molecular structure (the fifth molecular structure) that may replace the third molecular structure. For example, the answer 1921 may include at least one of the label M5 assigned to the fifth molecular structure, a molecular structure image 2115a of the fifth molecular structure, a name 2115b of the fifth molecular structure, and a description 2121a of why the fifth molecular structure may replace the third molecular structure.

Furthermore, the first region 2110 of the service page 1000 may include at least one of a fifth graphic object 2115 corresponding to the fifth molecular structure, the molecular structure image 2115a of the fifth molecular structure linked to the fifth graphic object 1914, a name 2115b of the fifth molecular structure, a description 2115c of the fifth molecular structure, a SMILES notation 2115d of the fifth molecular structure, and a property 2115e of the fifth molecular structure.

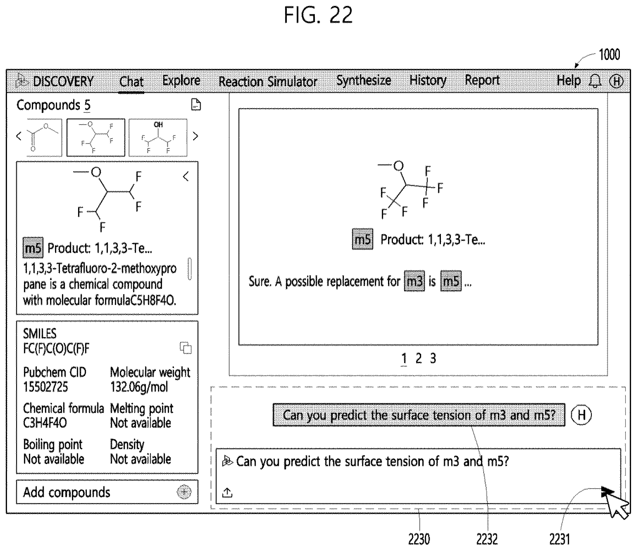

Next, the answer generation system 100 may receive a new user query including a label assigned to the fifth molecular structure through the third region of the service page 1000. For example, as illustrated in FIG. 22, the answer generation system 100 may receive a new user query 2232 (e.g., "Can you predict the surface tension of m3 and m5?") including the third label M3 assigned to the third molecular structure and the fifth label M5 assigned to the fifth molecular structure from the user terminal 10 based on a selection of a graphic object 2231 included in a third region 2230.

The answer generation system 100 may input the new user query 2232 to the ultra-large foundation model 200 based on receiving the new user query 2232 including the labels M3 and M5 assigned to the third molecular structure and the fifth molecular structure, respectively.

The ultra-large foundation model 200 may generate the answer to the new user query 2232 using at least a portion of the information on the third molecular structure corresponding to the third label M3, the fifth molecular structure corresponding to the fifth label M5, and the properties of the third molecular structure and the fifth molecular structure.

In this case, the ultra-large foundation model 200 may utilize at least one prediction model to generate the answer to the new user query 2232. For example, the ultra-large foundation model 200 may understand the contents included in the new user query 2232 and input the information on the properties of the third molecular structure, the fifth molecular structure, the third molecular structure, and the fifth molecular structure to the molecular property prediction model 500 based on the fact that the user query 2232 includes the contents "Can you predict the surface tension of m3 and m5?"

The molecular property prediction model 500 may predict the properties (e.g., surface tension) for the third molecular structure and the fifth molecular structure corresponding to the new user query 2232. The molecular property prediction model 500 may output the property prediction results of the third molecular structure and the fifth molecular structure as the output data.

The ultra-large foundation model 200 may determine the answer generation procedure performed for prediction corresponding to the new user query 1332 and the tool used in the answer generation procedure, and generate an answer 2321 (e.g., "surface tension of m3 is OO, surface tension of m5 is OO...,") to the user query 2232 using the determined answer generation procedure and tool, the output data of the molecular property prediction model 500, and the information on the third molecular structure and the fifth molecular structure (see FIG. 14).

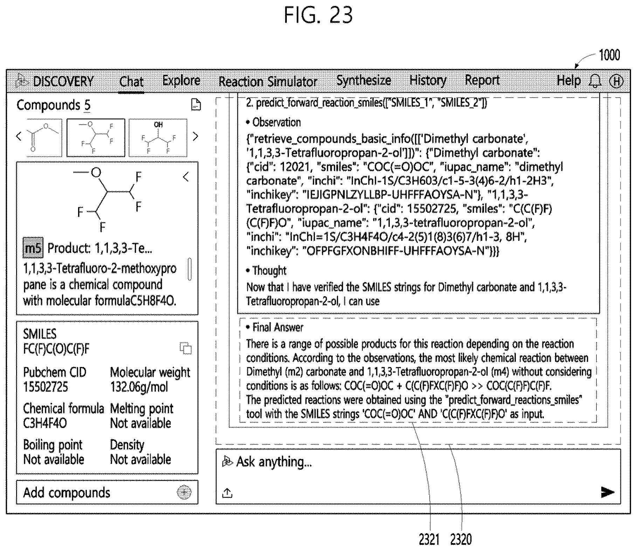

When the answer 2321 is generated, as illustrated in FIG. 23, the answer generation system 100 may provide the answer 2321 to the user query 2232 generated from the ultra-large foundation model 200 to a second region 2320 of the service page 1000. In this case, the answer generation system 100 may perform the update on the first region so that the information on the properties among the detailed information on the fifth molecular structure included in the first region is displayed corresponding to the predicted result.

Meanwhile, the present invention may provide a function for designing a molecule having the user's desired properties.

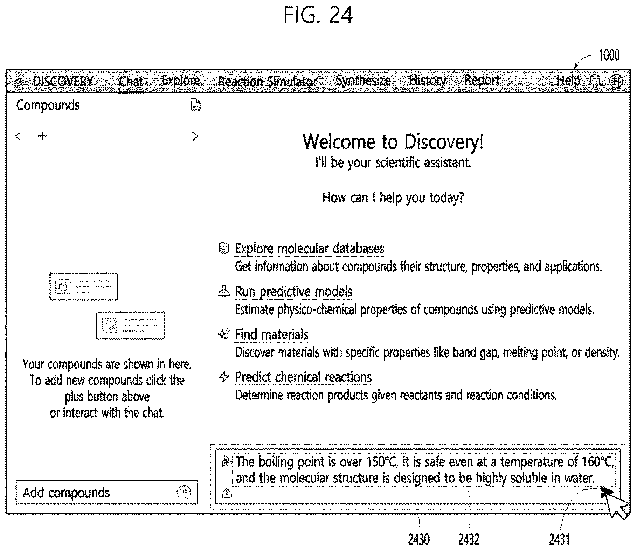

The answer generation system 100 may receive the user query including the information on the properties of the molecular structure for which the design is desired through the third area of the service page 1000. For example, as illustrated in FIG. 24, the answer generation system 100 may receive a user query 2432 (e.g., "The boiling point is over 150°C, it is safe even at a temperature of 160°C, and the molecular structure is designed to be highly soluble in water.") including information on at least one property of the molecular structure from the user terminal 10 based on the selection of a graphic object 2431 included in a third area 2530.

The answer generation system 100 may input the received user query 2432 to the ultra-large foundation model 200. The ultra-large foundation model 200 may input user query 2432 to the molecular property prediction model 500 based on the fact that the user query 2432 includes the contents "Design a molecular structure that is safe even at temperatures of 160°C with a boiling point of 150°C or higher and that dissolves well in water."

The molecular property prediction model 500 can design a specific molecular structure having the properties corresponding to the user query 2432, predict the properties of the designed specific molecular structure, and output the designed specific molecular structure and the property prediction results of the designed specific molecular structure as the output data. Hereinafter, for the convenience of description, the specific molecular structure having the physical properties corresponding to the user query 2432 is named "the first molecular structure."

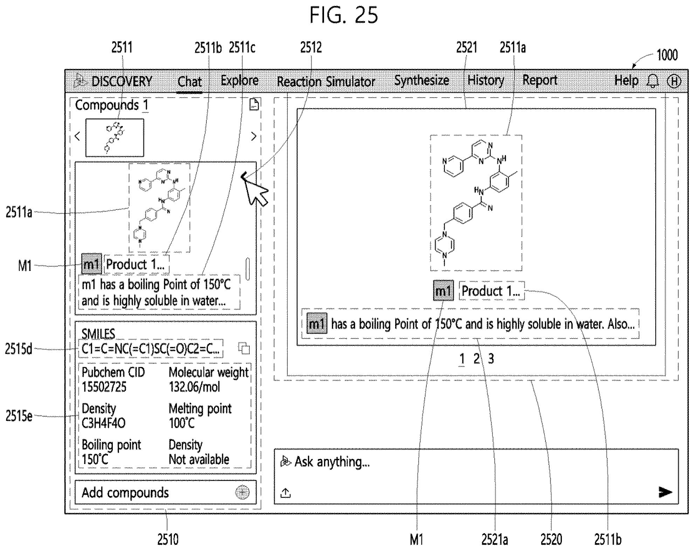

Meanwhile, the ultra-large foundation model 200 can generate an answer 2521 (e.g., "The boiling point of m1 is 150°C and it dissolves well in water...") to the user query 2432 using the output data of the molecular property prediction model 500 (see FIG. 25).

Here, the answer generation system 100 may perform the labeling on the first molecular structure so that a new label for specifying the first molecular structure is assigned based on the fact that the first molecular structure generated through the molecular property prediction model 500 is included in the answer 2521 to the user query 2432. For example, the answer generation system 100 may assign the first label M1 to the first molecular structure by performing the labeling on the first molecular structure. The first molecular structure and the first label M1 assigned to the first molecular structure may be stored in the pre-specified storage by being linked to the user account.

In addition, the answer generation system 100 may generate the first graphic object corresponding to the first molecular structure generated through the molecular property prediction model 500, and perform the update on the service page 1000 so that the first graphic object corresponding to the first molecular structure is included in one region of the service page 1000. For example, as illustrated in FIG. 25, the answer generation system 100 may perform the update on the first area 2510 so that the first graphic object 2511 is included in the first region 2510 of the service page 1000.

Furthermore, based on the update, the detailed information on the molecular structure corresponding to the first graphic object 2511 may be provided to the first region 2510 of the service page 1000 together with the first graphic object. For example, the first region 2510 may include a molecular structure image 2511a of the first molecular structure corresponding to the first graphic object 2511, a name 2511b of the first molecular structure, a description 2511c of the first molecular structure, a SMILES notation 2511d of the first molecular structure, and a property 2511e of the first molecular structure.

Meanwhile, the answer generation system 100 may provide the answer 2521 to the user query 2432 generated from the ultra-large foundation model 200 to the second region 2520 of the service page 1000.

The answer 2521 provided to the second area 2520 of the service page 1000 may include the contents related to the first molecular structure generated to correspond to the user query 2432. For example, the answer 2521 may include at least one of the first label M1 assigned to the first molecular structure, the molecular structure image 2511a of the first molecular structure, the name 2511b of the first molecular structure, and a description 2521a of the property of the first molecular structure.

Meanwhile, the answer generation system 100 may receive the editing request for the first molecular structure through the service page 1000.

A graphic object 2512 linked to the editing request receiving function for the first molecular structure may be provided to the first region 2510 of the service page 1000. For example, the answer generation system 100 may receive the editing request for the first molecular structure from the user terminal 10 based on the selection of the graphic object 2512.

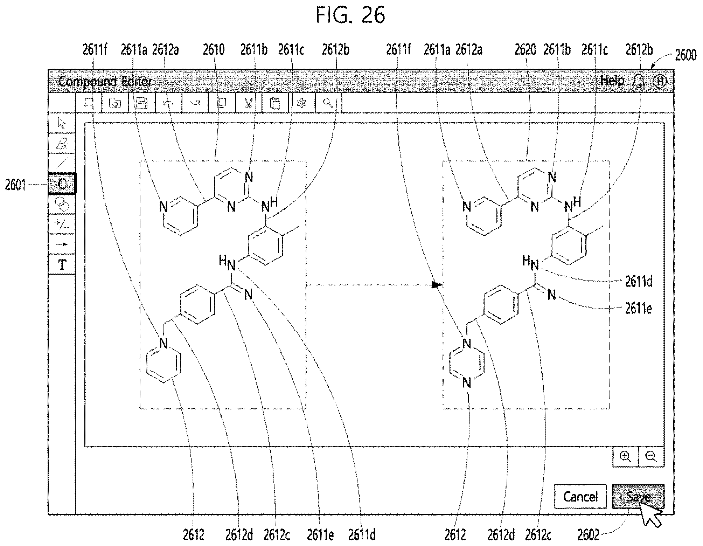

When the editing request is received, as illustrated in FIG. 26, the answer generation system 100 may provide an editing interface 2600 that provides the editing function for the first molecular structure. The editing interface 2600 may include a molecular structure image 2610 of the first molecular structure. The molecular structure image 2610 of the first molecular structure provided on the editing interface 2600 may include nodes 2611a, 2611b, 2611c, 2611d, 2611e, and 2611f corresponding to each of the atoms constituting the molecular structure, and edges 2612a, 2612b, 2612c, and 2612d indicating the bond relationship between the atoms.

As described above, the editing for the molecular structure may be the deletion or repositioning of at least one of nodes 2611a, 2611b, 2611c, 2611d, 2611e, and 2611f corresponding to each of the atoms constituting the molecular structure, and edges 2612a, 2612b, 2612c, and 2612d indicating the bond relationship between the atoms, or the addition of a new node corresponding to a new atom, or the addition of a new edge generating a new bond relationship between the atoms.

For example, the answer generation system 100 may activate a mode that may add a new node based on the selection of a graphic object 2601 linked to the addition function of a new node included in one region of the editing interface 2600. The answer generation system 100 may edit the image 2610 of the first molecular structure so that a new node 2611g is added to a location corresponding to the user input based on the fact that the user input for selecting a specific region 2612 of the image 2610 of the first molecular structure is received.

In this case, when the editing is performed on the first molecular structure as the editing target based on the user input, a molecular structure image 2620 corresponding to the edited molecular structure, which is different from the image 2610 of the first molecular structure before the editing, may be continuously displayed on the editing interface 2600. For example, based on the addition of a new node 2611g corresponding to the user selection, the molecular structure image 2620 to which the new node 2611g is added may be displayed on the editing interface 2600.

Furthermore, the answer generation system 100 may store the molecular structure 2620 (or molecular structure image) that has been edited (or has been edited) for the first molecular structure in the pre-specified storage by being linked to the user account. For example, the answer generation system 100 may generate the molecular structure 2620 in which the first molecular structure is edited based on a selection of a graphic object 2602 linked to an editing save (or completion) function included in the editing interface 2600 from the user terminal 10, and store the edited molecular structure 2620 in the pre-specified storage by linking the edited molecular structure 2620 to the user account.

Meanwhile, a new label for specifying the edited molecular structure may be assigned to the edited molecular structure 2620 (or the second molecular structure). The answer generation system 100 may perform the labeling on the edited molecular structure so that a new label for specifying the edited molecular structure is assigned. For example, the answer generation system 100 may perform the labeling on the edited molecular structure 2620 so that the second label M2, which is different from the first label M1 assigned to the first molecular structure before the editing, is assigned.

The answer generation system 100 may generate a second graphic object corresponding to the edited molecular structure 2620 and provide the generated second graphic object to one region of the service page 1000.

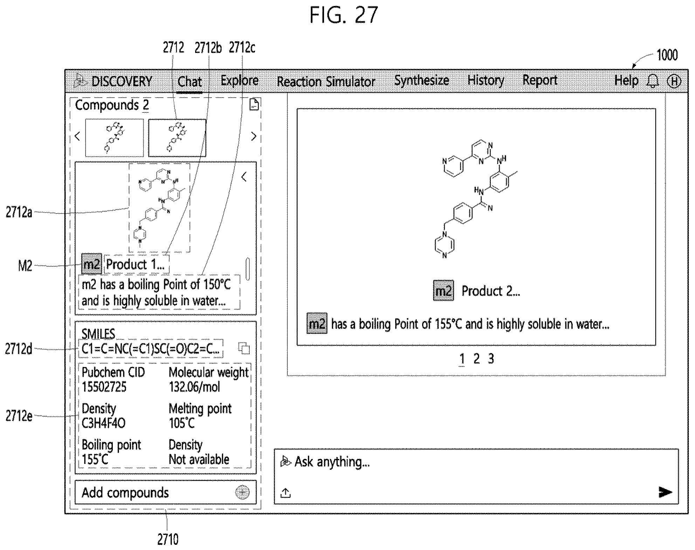

Specifically, as illustrated in FIG. 27, a second graphic object 2712 corresponding to the edited molecular structure 2620 may be provided to the first region 2710 (or a first sub-region) of the service page 1000. Here, the second graphic object 2712 corresponding to the edited molecular structure 2620 may include the molecular structure image of the edited molecular structure 2620.

In addition, the first region 2710 (or the first sub-region) of the service page 1000 may include a molecular structure image 2712a of the edited molecular structure 2620. In addition, the second label M2 assigned to the edited molecular structure 2620 may also be provided to the surrounding region of the molecular structure image 2712a.

Furthermore, the second graphic object 2712 corresponding to the edited molecular structure 2620 may further include the detailed information on the edited molecular structure 2620. For example, the graphic object 2712 may include at least one of a name 2712b of the edited molecular structure 2620, a description 2712c of the edited molecular structure 2620, a SMILES notation 2712d of the edited molecular structure 2620, and a property 2712e of the edited molecular structure 2620.

As described above, a new label (e.g., a second label M2) for specifying the edited molecular structure may be assigned to the edited molecular structure 2620.

When the answer generation system 100 receives a user query including the edited molecular structure 2620 to which the second label M2 is assigned, the answer generation system 100 may input the received user query to the ultra-large foundation model 200.

The ultra-large foundation model 200 may generate the answer using the edited molecular structure 2620 included in the user query. More specifically, the ultra-large foundation model 200 may generate the answer to the user query using the edited molecular structure corresponding to the second label. More specific details related to the answer generation process have been described above, and therefore, the answer generation process will be described briefly.

Furthermore, the answer generation system 100 may provide the answer generated from the ultra-large foundation model 200 to the user terminal 10. For example, the answer generation system 100 may provide the answer to a user query including the edited molecular structure in the second region of the service page 1000.

Meanwhile, as described above, the user may have the user account pre-registered in the answer generation system 100 according to the present invention.

Accordingly, various types of information related to the user account may be stored in the storage unit 140 of the answer generation system 100. Here, the information related to the user account may include at least one of the user's (or user account's) history information and user's metadata (e.g., name, gender, age, major, occupation, workplace (or company), etc.).

More specifically, the user's history information may include information related to various events that are performed in the user account. For example, the events that have been performed in the user account may include at least one of: i) inputting a user query to acquire an answer from the ultra-large foundation model 200, ii) inputting (or selecting) a document, iii) inputting a new (or additional) query for an answer generated from the ultra-large foundation model 200.

Based on these events, the user's history information may include at least one of: i) the user query input by the user, ii) the document information (or user's document input history) input by the user, iii) content (e.g., a specific molecular structure and information related to the specific molecular structure) extracted from a document input by the user, and iv) an answer from the ultra-large foundation model 200 to the user query.

Accordingly, the storage unit 140 of the answer generation system 100 may store at least one of the analysis target document, the extracted molecular structure, the label for (or assigned to) the extracted molecular structure, the user query, and the answer to the user query by being linked to the user account.

Here, the information related to the extracted molecular structure may be i) the information extracted from the analysis target document, or ii) the information extracted from the user query. Alternatively, the information related to the extracted molecular structure may be i) the information generated from the chemical reaction prediction model 400, or ii) the information generated from the molecular property prediction model 500.

In this regard, the analysis target document and the information on the molecular structure extracted from the analysis target document may be matched and stored in the storage unit 140. For example, as illustrated in FIG. 28, the storage unit 140 may store a plurality of molecular structures 2811, 2812, 2813, 2821, and 2822 extracted from different first analysis target document 2810 and second analysis target document 2820, respectively.

In addition, labels for specifying each of the plurality of molecular structures 2811, 2812, 2813, 2821, and 2822 may be assigned to each of the plurality of molecular structures 2811, 2812, 2813, 2821, and 2822. The information on the labels assigned to each of the plurality of molecular structures 2811, 2812, 2813, 2821, and 2822 and each of the plurality of molecular structures 2811, 2812, 2813, 2821, and 2822 may be stored in the storage unit 140 by being linked to a user account U.

Meanwhile, in order for the user to be able to use molecular structures extracted from a plurality of different documents rather than a single document, information that may distinguish molecular structures including the same label or the same meaning among the molecular structures extracted from the plurality of different documents should be assigned.

In the present invention, a user environment that allows the user to use various pieces of information extracted from each of the different analysis target documents together may be provided.

To this end, in the present invention, the labeling may be performed on each of the different documents stored in the storage unit 140, so that labels may be assigned to each of the different documents. For example, a first label D1 may be assigned to the first analysis target document 2810, and a second label D2 may be assigned to the second analysis target document 2820. Here, it may be confirmed that the information on the label D assigned to the analysis target document and the label m assigned to the molecular structure is different from each other. In addition, the information on the labels assigned to each of the different documents 2810 and 2820 may be stored in the storage 140 by being linked to the user account U.

The user may input at least one of the labels M1 and M2 corresponding to each of the different documents 2810 and 2820 stored in the storage 140 and the labels assigned to each of the plurality of molecular structures 2811, 2812, 2813, 2821, and 2822 to the user query answer generation system 100 to obtain an answer from the ultra-large foundation model 200. The answer generation system 100 may receive the user query as the input, generate the answer to the user query, and provide the generated answer to the user account U (or user terminal).

In an embodiment, a user may input a user query (e.g., "Extract molecular structure from D1") for extracting the molecular structure from the first analysis target document 2810 to the answer generation system 100. The answer generation system 100 may extract the molecular structure from the first analysis target document 2810 in response to the user query, generate the answer (e.g., molecular structures extracted from D1 include m1, m2, m3, etc.) including the extracted molecular structure, and provide the generated answer to the user account U.

In another embodiment, a user may receive a user query (e.g., predict the chemical reaction of m1 of D1 and m2 of D2) for predicting a chemical reaction of the first molecular structure 2811 extracted from the first analysis target document 2810 and the first molecular structure 2821 extracted from the second analysis target document 2820. The answer generation system 100 may generate an answer (e.g., m3 is generated through the chemical reaction of m1 of D1 and m2 of D2...) including a predicted result of a chemical reaction of the first molecular structure 2811 extracted from the first analysis target document 2810 and the first molecular structure 2821 extracted from the second analysis target document 2820 in response to the user query and provide the answer to the user account U.

That is, by assigning a unique label to each of various documents, even if the molecular structure to which the same label is assigned appears repeatedly in multiple documents, it is possible to generate the answer to the user query without confusion. The user may quickly access the information he/she needs by using the labels assigned to each of the various documents, generate a query using various documents, and receive the answer to the query.

Furthermore, in the present invention, the labels related to the extracted molecular structure are systematically assigned and stored, so the user may easily access information he/she needs or use the information to generate the answer to the user query.

As described above, the storage unit 140 of the answer generation system 100 may store at least one of the analysis target document, the extracted molecular structure, the label for (or assigned to) the extracted molecular structure, the user query, and the answer to the user query by being linked to the user account U.

The answer generation system 100 may perform clustering on various pieces of information (or history information) stored in linkage with the user account U based on various criteria. Here, the clustering can mean grouping data with similar characteristics into one cluster (or cluster or group) and separating data with different characteristics into different clusters.

In the present invention, the criteria for the clustering may be set in various ways. For example, when performing the clustering on the extracted molecular structures, various criteria may include at least one of i) the shape or arrangement of the molecular structures, ii) the chemical reaction or chemical properties (e.g., acidity, alkalinity, polarity, etc.) of the molecular structure, iii) the properties of the molecular structure, and iv) the use cases and application fields of the molecular structure.

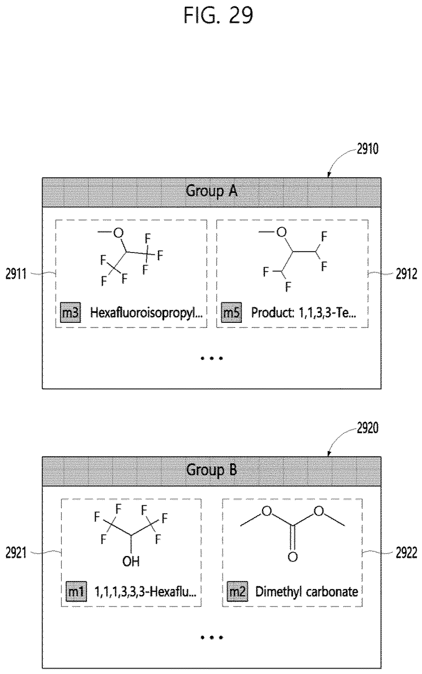

In an embodiment, as illustrated in FIG. 29, the answer generation system 100 may perform the clustering on the extracted molecular structures 2911, 2912, 2921, and 2922 based on the property of the extracted molecular structures 2911, 2912, 2921, and 2922. In this case, the first group 2910 may include the first molecular structure 2911 and the second molecular structure 2912 having high boiling points (e.g., 150°C or higher), and the second group 2920 may include the third molecular structure 2921 and the fourth molecular structure 2922 having low boiling points (e.g., 100°C or lower).

In this case, the extracted molecular structures 2911, 2912, 2921, and 2922 may be information extracted or generated using at least one of the analysis target document, the pre-trained prediction model, the user query, and the answer to the user query, and it may be understood that such information is grouped through the clustering.

In addition, the criteria for the clustering in the present invention may be set based on the user query and the answer to the user query. For example, when performing the clustering based on the user query and the answer to the user query, the criteria for the clustering may include a topic of the user query or keywords included in the user query and the answer to the user query.

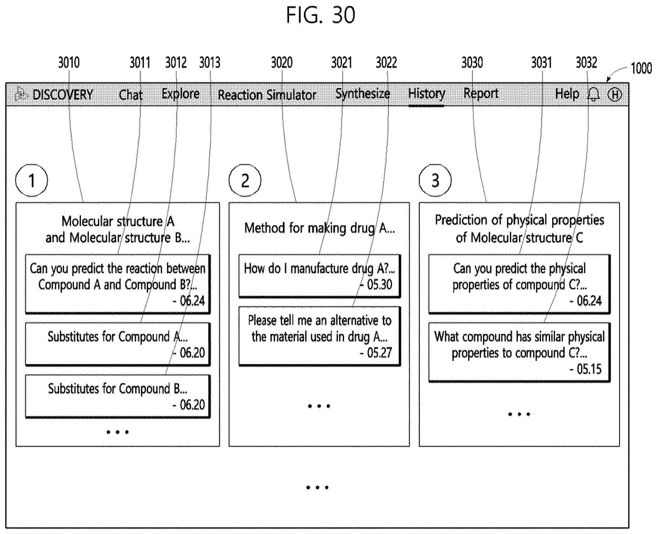

In an embodiment, as illustrated in FIG. 30, the answer generation system 100 may perform the clustering on the user query and the answer to the user query based on the keywords included in the user query and the answer to the user query. In this case, a first group 3010 may include user queries and answers 3011, 3012, and 3013 having keywords related to the chemical reaction prediction of the molecular structure, a second group 3020 may include user queries and answers 3021 and 3022 having keywords related to a new material design, and a third group 3030 may include the user queries and answers 3031 and 3032 having keywords related to the property prediction of the molecular structure.

That is, in the present invention, by grouping similar data through the clustering, data may be systematically managed, and more accurate and relevant answers may be provided to the user query.

In an embodiment, the answer generation system 100 may quickly extract information corresponding to the user request based on the clustered data, thereby providing a personalized service to the user.

Meanwhile, in the present invention, the report related to the user account U may be generated using various pieces of information linked to the user account U.

In an embodiment, among various pieces of information linked to the user account U, the information used for generating the report may be a chat list (or chat list) including the user query and the answer to the user query. However, the information used for generating the report is not necessarily limited thereto, and various pieces of information may be used for generating the report in addition to ones mentioned. Hereinafter, for the convenience of description, a method of generating a report will be described using the chat list.

The answer generation system 100 may provide at least one chat list including the user query and the answer to the user query through the service page 1000. For example, as illustrated in FIG. 31, the answer generation system 100 may provide, to one region of the service page 1000, a plurality of graphic objects 3011, 3012, 3013, 3014, 3015, 3016, and 3017 each corresponding to the plurality of chat lists including queries and answers of different contents.

In this case, each of the plurality of graphic objects 3011, 3012, 3013, 3014, 3015, 3016, and 3017 may be provided by being sorted according to metadata matched to each of the plurality of chat lists. For example, each of the plurality of graphic objects 3011, 3012, 3013, 3014, 3015, 3016, and 3017 may be provided by being sorted based on a date or time matching each of the plurality of chat lists.

The answer generation system 100 may specify at least one chat list to be used for generating the report based on the user input. For example, the answer generation system 100 may specify a first chat list corresponding to the first graphic object 3011 as the chat list to be used for generating the report from the user terminal 10 based on the selection of the first graphic object 3011 from among the plurality of graphic objects 3011, 3012, 3013, 3014, 3015, 3016, and 3017 and the selection of the graphic object 3101 linked to the report generation request function.

In this case, the chat list may include various pieces of information (e.g., molecular structure, label assigned to molecular structure, detailed information related to molecular structure, etc.) extracted from at least one of the analysis target document, the user query, the answer of the ultra-large foundation model 200, and the answer of the ultra-large foundation model 200.

Furthermore, the answer generation system 100 may input a specific chat list to ultra-large foundation model 200 and generate the report on the specific chat list. For example, as illustrated in FIG. 32, the ultra-large foundation model 200 may receive a specific first chat list as an input and specify contents (or content) related to molecular structure among the information included in the first chat list. Then, the ultra-large foundation model 200 may generate a report 3200 on the first chat list using the specified contents.

In an embodiment, the answer generation system 100 may provide the report 3200 generated for the first chat list to the user terminal 10 through the service page 1000. In addition, the answer generation system 100 may store the report 3200 for the first chat list in the storage unit 140 by linking the report 3200 to the user account U.

In this way, the user may receive a customized report automatically generated without having to write the report directly, which may greatly save the user's time and effort.

As described above, according to the answer generation method and system of the ultra-large foundation model according to the present invention, by generating and providing the answer suitable for the user's query based on the data extracted from the document, it is possible for the user to minimize the risk of research failure by receiving suggestions for the optimal research method.

In addition, according to the answer generation method and system of the ultra-large foundation model according to the present invention, it is possible to provide the answer to the user query using the data that is extracted from the document or generated from the pre-trained prediction model. Accordingly, the user can quickly and accurately be provided with his/her required information and reduce the time and/or cost of research and/or development.

Furthermore, according to the answer generation method and system of the ultra-large foundation model according to the present invention, it is possible to generate the answer to the user query using the predicted results from the pre-trained prediction model and provide the generated answer to the user. Accordingly, it is possible for the user to shorten the time required for research and/or development and reduce the number of trial and errors in research and/or development.

Furthermore, according to the answer generation method and system of the ultra-large foundation model according to the present invention, by visualizing and providing the extracted molecular structure and related data through the user interface, it is possible for the user to intuitively recognize his/her required information and understand the information more quickly, thereby increasing the accuracy and efficiency of the research.

Meanwhile, the present invention described above may be implemented as a program that is executed by one or more processes on a computer and can be stored on a computer-readable medium (or recording medium).

Furthermore, the present invention described above can be implemented as a computer-readable code or command on a medium in which a program is recorded. The present invention may be provided in the form of a program.

Meanwhile, the computer-readable medium may include all kinds of recording devices in which data that may be read by a computer system are stored. Examples of the computer-readable medium include a hard disk drive (HDD), a solid state disk (SSD), a silicon disk drive (SDD), a read only memory (ROM), a random access memory (RAM), a compact disk (CD)-ROM, a magnetic tape, a floppy disk, an optical data storage device, and the like.

Furthermore, the computer-readable medium may include a storage and may be a server or a cloud storage that an electronic device may access through communication. In this case, the computer may download the program according to the present invention from the server or cloud storage through wired or wireless communication.

Furthermore, in the present invention, the computer described above is an electronic device equipped with a processor, that is, a central processing unit (CPU), and the type of electronic device is not particularly limited.

Meanwhile, the above-described detailed description is to be interpreted as being illustrative rather than being restrictive in all aspects. The scope of the present invention is to be determined by reasonable interpretation of the claims, and all modifications within an equivalent range of the present invention fall in the scope of the present invention.

## Claims

1. An answer generation method performed by cooperation of a memory and at least one processor, the answer generation method comprising:
specifying an analysis target document;
extracting a plurality of content from the document;
storing the plurality of content extracted from the document in the memory;
receiving a user query from a user terminal;
specifying specific content related to the user query among the plurality of content stored in the memory;
processing the specific content as input to a pre-trained chemical reaction prediction model; and
generating an answer to the user query using output data of the chemical reaction prediction model.

2. The answer generation method of claim 1, further comprising:
performing labeling so that a label is assigned to at least some of the plurality of content; and
providing a graphic object corresponding to each content to which the label is assigned to a region of a service page where the user query is received.

3. The answer generation method of claim 2, further comprising:
analyzing a relationship between the plurality of content based on a meaning of each of the plurality of content; and
grouping related content among the plurality of content based on the relationship,
wherein, in the performing of the labeling, the same label is assigned to the grouped content through the grouping.

4. The answer generation method of claim 3, wherein in the extracting of the plurality of content, the plurality of content satisfying a preset content criterion are extracted using a document understanding model.

5. The answer generation method of claim 4, wherein the preset content criterion is related to contents related to a molecular structure related to at least one of chemistry, bio, new materials, new substances, and new drug development.

6. The answer generation method of claim 4, wherein, in the document understanding model, at least one of a text, a molecular structure, a formula, a chart, a table, and an image satisfying the preset contents is extracted from the document as the plurality of contents.

7. The answer generation method of claim 6, wherein, in the grouping, contents for the same molecular structure among at least one of the text, the molecular structure, the formula, the chart, the table, and the image extracted from the plurality of content are grouped as the related content.

8. The answer generation method of claim 7, wherein the grouped content includes at least one of a molecular structure image, a name, a property, and a string according to a SMILES notation of a specific molecular structure corresponding to the grouped content,

9. The answer generation method of claim 8, wherein at least some of the content included in the grouped content for the specific molecular structure is generated by at least one of the ultra-large foundation model, the pre-trained chemical reaction prediction model, and the pre-trained molecular property prediction model.

10. The answer generation method of claim 8, wherein, in the specifying of the specific content,
the user query is analyzed to extract a label indicating the grouped content from the query,
specific grouped content corresponding to the label is specified, and
a molecular structure of the specific grouped content is processed as input to the prediction model, and
in the generating of the answer, the answer is generated using output data of the prediction model and contents constituting the grouped content.

11. The answer generation method of claim 10, wherein the generating of the answer to the user query includes:
determining an answer generation procedure performed for prediction corresponding to the user query and a tool used in the answer generation procedure;
providing information on the determined answer generation procedure and tool to the service page; and
generating the answer to the user query using the determined answer generation procedure and tool.

12. The answer generation method of claim 2, wherein, in the extracting of the plurality of content, contents related to a molecular structure related to at least one of chemistry, bio, new materials, new substances, and new drug development are extracted from the document, and
the content to which the label is assigned is content related to the molecular structure extracted from the document, and
the one region includes a graphic object corresponding to the extracted molecular structure.

13. The answer generation method of claim 12, wherein the one region includes a plurality of graphic objects each corresponding to a plurality of molecular structures when the plurality of molecular structures are extracted from the document,
a first graphic object among the plurality of graphic objects includes an image of a first molecular structure corresponding to the first graphic object among the plurality of molecular structures, and
a second graphic object among the plurality of graphic objects includes an image of a second molecular structure corresponding to the second graphic object among the plurality of molecular structures.

14. The answer generation method of claim 13, wherein the document is provided to another region distinguished from the one region of the service page, and
highlighting objects overlap with a first region including the first molecular structure of the document provided to the service page and a second region including the second molecular structure, respectively, so that it is identified that the first molecular structure and the second molecular structure were extracted from the document.

15. The answer generation method of claim 14, wherein, in the first region, a first label assigned to correspond to the first molecular structure is provided around a first highlighting object overlapping with the first region, and
in the second region, a second label assigned to correspond to the second molecular structure is provided around a second highlighting object overlapping with the second region.

16. The answer generation method of claim 12, further comprising:
providing detailed information on a graphic object selected according to the user input to the service page based on receiving user input for selecting one of the plurality of graphic objects,
wherein the detailed information includes at least one of a molecular structure image of a specific molecular structure corresponding to the selected graphic object, a name of the molecular structure, a description of the molecular structure, a property of the molecular structure, and a SMILES notation of the molecular structure.

17. An answer generation system, comprising:
a memory and at least one processor,
wherein the memory and the processor cooperate to
specify an analysis target document,
extract a plurality of content from the document,
receive a user query from a user terminal, and
specify specific content related to the user query among the plurality of content, and
the specific content is processed as input to a pre-trained prediction model, and generates an answer to the user query using output data of the prediction model.

18. A program stored on a computer-readable recording medium, executable by one or more processes on an electronic device, the program comprising instructions to execute:
specifying an analysis target document;
extracting a plurality of content from the document;
receiving a user query from a user terminal;
specifying specific content related to the user query among the plurality of content;
processing the specific content as input to a pre-trained prediction model; and
generating an answer to the user query using output data of the prediction model.
